# EUROPEAN PATENT SPECIFICATION

(11) **EP 3 265 115 B1**
(45) Date of publication and mention of the grant of the patent: **22.06.2022**
(21) Application number: 16759540.4
(22) Date of filing: 03.03.2016
(51) Int. Cl.: A61K 38/29, A61K 38/16, A61P 19/08, A61P 19/10, C07K 14/47, A61K 31/663

(54) **ABALOPARATIDE COMBINED WITH ALENDRONATE FOR REDUCING NON-VERTEBRAL BONE FRACTURES**
ABALOPARATID KOMBINIERT MIT ALENDRONAT ZUR VERRINGERUNG DER FRAKTUREN VON NICHT VERTEBRALEN KNOCHEN
ABALOPARATIDE COMBINÉ AVEC ALENDRONATE POUR LA RÉDUCTION DE FRACTURES OSSEUSES NON VERTÉBRALES

(30) Priority: 03.03.2015 US 201562127729 P; 22.05.2015 US 201562165841 P; 05.08.2015 US 201562201564 P; 09.10.2015 US 201562239733 P; 14.01.2016 US 201662278762 P
(43) Date of publication of application: 10.01.2018
(73) Proprietor: Radius Health, Inc., Boston MA 02210 (US)
(72) Inventor: HATTERSLEY, Gary, Stow, Massachusetts 01775 (US)
(74) Representative: Carpmaels & Ransford LLP
(86) International application number: PCT/US2016/020787
(87) International publication number: WO 2016/141250

(56) References cited:
- WO-A1-2009/137093
- WO-A1-2010/022176
- WO-A1-2014/004465
- WO-A1-2017/062922
- WO-A2-2012/145665
- US-A1- 2010 016 223
- BENJAMIN Z. LEDER ET AL: "Effects of Abaloparatide, a Human Parathyroid Hormone-Related Peptide Analog, on Bone Mineral Density in Postmenopausal Women with Osteoporosis", JOURNAL OF CLINICAL ENDOCRINOLOGY AND METABOLISM, vol. 100, no. 2, 1 February 2015 (2015-02-01), pages 697-706, XP055505823, US ISSN: 0021-972X, DOI: 10.1210/jc.2014-3718
- DATABASE EMBASE [Online] ELSEVIER SCIENCE PUBLISHERS, AMSTERDAM, NL; 9 October 2015 (2015-10-09), DOHMEIER D ET AL: "Transdermal delivery of abaloparatide: Optimization of the pharmacokinetic profile in cynomologus monkeys", XP002784613, Database accession no. EMB-620769961 & JOURNAL OF BONE AND MINERAL RESEARCH 20150201 JOHN WILEY AND SONS INC. NLD, vol. 30, no. Supplement 1, 17 December 2015 (2015-12-17), ISSN: 1523-4681
- DATABASE EMBASE [Online] ELSEVIER SCIENCE PUBLISHERS, AMSTERDAM, NL; 9 October 2015 (2015-10-09), HARRIS A ET AL: "Response rates for hip, femoral neck and lumbar spine BMD are higher for patients treated with abaloparatide when compared to placebo or teriparatide - Results of the ACTIVE trial", XP002784614, Database accession no. EMB-620769884 & JOURNAL OF BONE AND MINERAL RESEARCH 20150201 JOHN WILEY AND SONS INC. NLD, vol. 30, no. Supplement 1, 17 December 2015 (2015-12-17), ISSN: 1523-4681
- DATABASE EMBASE [Online] ELSEVIER SCIENCE PUBLISHERS, AMSTERDAM, NL; 9 October 2015 (2015-10-09), FITZPATRICK L ET AL: "Effects of abaloparatide on major osteoporotic fracture incidence in postmenopausal women with osteoporosis - Results of the Phase 3 ACTIVE trial", XP002784615, Database accession no. EMB-620769127 & JOURNAL OF BONE AND MINERAL RESEARCH 20150201 JOHN WILEY AND SONS INC. NLD, vol. 30, no. Supplement 1, 17 December 2015 (2015-12-17), ISSN: 1523-4681
- DATABASE EMBASE [Online] ELSEVIER SCIENCE PUBLISHERS, AMSTERDAM, NL; 9 October 2015 (2015-10-09), COSMAN F: "Eighteen months of treatment with abaloparatide followed by six months of treatment with alendronate in postmenopausal women with osteoporosis - Results of the ACTIVExtend trial", XP002784616, Database accession no. EMB-620768919 & JOURNAL OF BONE AND MINERAL RESEARCH 20150201 JOHN WILEY AND SONS INC. NLD, vol. 30, no. Supplement 1, 17 December 2015 (2015-12-17), ISSN: 1523-4681
- BRIGITTE UEBELHART ET AL: "Maladies osseusses", REV MED SUISSE, vol. 12, no. 500, 13 January 2016 (2016-01-13), pages 49-54, XP055505897,
- MAKRAS POLYZOIS ET AL: "Novel therapies for osteoporosis", METABOLISM, CLINICAL AND EXPERIMENTAL, vol. 64, no. 10, 21 July 2015 (2015-07-21) , pages 1199-1214, XP029269396, ISSN: 0026-0495, DOI: 10.1016/J.METABOL.2015.07.011
- PAUL D. MILLER ET AL: "Effects of Abaloparatide on Vertebral and Non-Vertebral Fracture Incidence in Postmenopausal Women with Osteoporosis - Results of the Phase 3 Active Trial : New Discoveries in Bone Health and Disease", ENDOCRINE SOCIETY'S 97TH ANNUAL MEETING AND EXPO, 5 March 2015 (2015-03-05), pages 1-2, XP055506001,
- Anonymous: "Radius Announces Positive Phase 3 Top-Line Results - MPM Capital", , 22 December 2014 (2014-12-22), pages 1-7, XP055506014, Retrieved from the Internet: URL:http://www.mpmcapital.com/press/radius -announces-positive-phase-3-top-line-resul ts/ [retrieved on 2018-09-11]
- Anonymous: "Radius Announces Positive Phase 3 Top-Line Results for Its Investigational Drug Abaloparatide-SC in Postmenopausal Women With Severe Osteoporosis | Radius Health, Inc.", , 22 December 2014 (2014-12-22), pages 1-8, XP055506045, Retrieved from the Internet: URL:http://ir.radiuspharm.com/news-release s/news-release-details/radius-announces-po sitive-phase-3-top-line-results-its?Releas eID=888584 [retrieved on 2018-09-11]
- FELICIA COSMAN: "Abaloparatide: a new anabolic therapy on the horizon", BONEKEY REPORTS, vol. 4, 29 April 2015 (2015-04-29), XP055506062, DOI: 10.1038/bonekey.2015.28
- SERGE FERRARI: "Future directions for new medical entities in osteoporosis", BAILLIERE'S BEST PRACTICE AND RESEARCH. CLINICAL ENDOCRINOLOGYAND METABOLISM, vol. 28, no. 6, 20 August 2014 (2014-08-20), pages 859-870, XP055579227, GB ISSN: 1521-690X, DOI: 10.1016/j.beem.2014.08.002
- PAUL MILLER ET AL: "Treatment with Abaloparatide Significantly Reduces Wrist Fractures Compared to Teriparatide - Results of the Phase 3 ACTIVE Trial", ECTS-IBMS 2015, 28 April 2015 (2015-04-28), pages 1-4, XP055579321,
- L. OEI ET AL: "High Bone Mineral Density and Fracture Risk in Type 2 Diabetes as Skeletal Complications of Inadequate Glucose Control: The Rotterdam Study", DIABETES CARE, vol. 36, no. 6, 11 January 2013 (2013-01-11), pages 1619-1628, XP055579816, US ISSN: 0149-5992, DOI: 10.2337/dc12-1188
- OEI LING ET AL: "Diabetes, Diabetic Complications, and Fracture Risk", CURRENT OSTEOPOROSIS REPORTS, CURRENT SCIENCE INC, US, vol. 13, no. 2, 4 February 2015 (2015-02-04), pages 106-115, XP035465158, ISSN: 1544-1873, DOI: 10.1007/S11914-015-0260-5 [retrieved on 2015-02-04]
- JANINA M PATSCH ET AL: "Increased cortical porosity in type 2 diabetic postmenopausal women with fragility fractures", JOURNAL OF BONE AND MINERAL RESEARCH, vol. 28, no. 2, 15 January 2013 (2013-01-15), pages 313-324, XP055309972, US ISSN: 0884-0431, DOI: 10.1002/jbmr.1763
- TEICHMANN, J ET AL.: 'Increase in Bone Mineral Density of Patients with Rheumatoid Arthritis Treated With Anti-TNF-alpha Antibody: a Prospective Open-Label Pilot Study.' RHEUMATOLOGY. vol. 44, no. 12, 01 November 2005, pages 1546 - 1548, XP002551323
- PATSCH, JM ET AL.: 'Increased Cortical Porosity in Type-2 Diabetic Postmenopausal Women with Fragility Fractures.' JOURNAL OF BONE AND MINERAL RESEARCH. vol. 28, no. 2, February 2013, pages 313 - 324, XP055309972
- Anonymous: "NCT01343004: Study to Evaluate the Safety and Efficacy of BA058 (Abaloparatide) for Prevention of Fracture in Postmenopausal Women (ACTIVE)", , 26 January 2015 (2015-01-26), pages 1-6, XP055706128, Retrieved from the Internet: URL:https://clinicaltrials.gov/ct2/history /NCT01343004?V_13=View#StudyPageTop [retrieved on 2020-06-17]
- Anonymous: "FOSAMAX - HIGHLIGHTS OF PRESCRIBING INFORMATION", , 1 February 2012 (2012-02-01), pages 1-24, XP055802143, Retrieved from the Internet: URL:http://web.archive.org/web/20130824100 835if_/http://www.accessdata.fda.gov/drugs atfda_docs/label/2012/021575s017lbl.pdf [retrieved on 2021-05-06]
- DATABASE EMBASE [Online] ELSEVIER SCIENCE PUBLISHERS, AMSTERDAM, NL; 1 February 2015 (2015-02-01), COSMAN F: "Eighteen months of treatment with abaloparatide followed by six months of treatment with alendronate in postmenopausal women with osteoporosis - Results of the ACTIVExtend trial", Database accession no. EMB-620768919 & JOURNAL OF BONE AND MINERAL RESEARCH 20150201 JOHN WILEY AND SONS INC. NLD, vol. 30, no. Supplement 1, 1 February 2015 (2015-02-01), ISSN: 1523-4681
- DATABASE EMBASE [Online] ELSEVIER SCIENCE PUBLISHERS, AMSTERDAM, NL; 1 February 2015 (2015-02-01), DOHMEIER D ET AL: "Transdermal delivery of abaloparatide: Optimization of the pharmacokinetic profile in cynomologus monkeys", Database accession no. EMB-620769961 & JOURNAL OF BONE AND MINERAL RESEARCH 20150201 JOHN WILEY AND SONS INC. NLD, vol. 30, no. Supplement 1, 1 February 2015 (2015-02-01), ISSN: 1523-4681
- DATABASE EMBASE [Online] ELSEVIER SCIENCE PUBLISHERS, AMSTERDAM, NL; 1 February 2015 (2015-02-01), HARRIS A ET AL: "Response rates for hip, femoral neck and lumbar spine BMD are higher for patients treated with abaloparatide when compared to placebo or teriparatide - Results of the ACTIVE trial", Database accession no. EMB-620769884 & JOURNAL OF BONE AND MINERAL RESEARCH 20150201 JOHN WILEY AND SONS INC. NLD, vol. 30, no. Supplement 1, 1 February 2015 (2015-02-01), ISSN: 1523-4681
- DATABASE EMBASE [Online] ELSEVIER SCIENCE PUBLISHERS, AMSTERDAM, NL; 1 February 2015 (2015-02-01), FITZPATRICK L ET AL: "Effects of abaloparatide on major osteoporotic fracture incidence in postmenopausal women with osteoporosis - Results of the Phase 3 ACTIVE trial", Database accession no. EMB-620769127 & JOURNAL OF BONE AND MINERAL RESEARCH 20150201 JOHN WILEY AND SONS INC. NLD, vol. 30, no. Supplement 1, 1 February 2015 (2015-02-01), ISSN: 1523-4681

## Description

### BACKGROUND

As our population ages, osteoporotic fractures are expected to have an increasing impact on the health of our population. Today, osteoporosis is estimated to affect over 20 million Americans, with 1.5 million osteoporotic fractures occurring in the United States every year (1). In patients with established osteoporosis, currently available medications can only modestly decrease the risk of clinical non-vertebral fracture (2, 3). At present, the mainstay of osteoporosis treatment is the use of oral and intravenous bisphosphonates. These drugs act by suppressing bone resorption but also decrease bone formation (4). Teriparatide (TPTD, hPTH(1-34)) is the only currently-available anabolic agent, and it acts by a mechanism that involves stimulating new bone formation (along with resorption) and reconstituting internal bone microarchitecture (5-7). The effects of teriparatide on bone mineral density (BMD) are superior to antiresorptive agents at the spine, but its effects at the hip are more modest, and often delayed until the second year of a 2-year course of therapy (8, 9). As hip fractures are particularly common among osteoporosis patients, there is a need to develop new treatments for improvement of BMD and decrease of hip fracture risk in osteoporosis patients.

Furthermore, patients with a high cortical porosity may have higher risk of fracture, even with slightly reduced or normal BMD (10). Thus, there is also a need to develop new treatment for not only improving BMD but also the microarchitecture of the bones to reduce fracture risk.

A press release dated 21 December 2014 (http://ir.radiuspharm.com/news-releases/newsrelease-details/radius-announces-positive-phase-3-top-line-results-its?ReleaseID=888584) and a press release dated 22 December 2014 (http://www.mpmcapital.com/press/radius-announcespositive-phase-3-top-line-results) relate to top-line results for abaloparatide-SC in postmenopausal women with severe osteoporosis.

### SUMMARY

The invention provides a polypeptide of SEQ ID NO:1 and alendronate for use in preventing or reducing non-vertebral bone fractures in a subject in need thereof, wherein said use comprises administering to the subject the polypeptide once daily by subcutaneous injection for a first period of time of 18 months, followed by an administration of alendronate for six months.

The·P·THrP analogue for use in the present invention is abaloparatide ([Glu^{22, 25}, Leu^{23, 28, 31}, Aib²⁹, Lys^{26, 30}]hPTHrP(1-34)NH₂), which has the amino acid sequence set forth in SEQ ID NO:1:.

Ala Val Ser Glu His Gln Leu Leu His Asp Lys Gly Lys Ser Ile Gln Asp Leu Arg Arg

Arg Glu Leu Leu Glu Lys Leu Leu Aib Lys Leu His Thr Ala. Aib is a-aminoisobutyric acid or 2-aminoisobutyric acid.

In certain embodiments, the subject has diabetes (e.g., type II diabetes). In certain embodiments, the subject has osteoporosis.

The abaloparatide and alendronate for use provided herein comprises administering to the subject a therapeutically effective amount of abaloparatide and alendronate.

The PTHrP analogue for use of the invention is abaloparatide. In certain embodiments, the non-vertebral bone fractures are hip or wrist fractures. The method comprises administering to the subject a therapeutically effective amount of the anti-resorptive agent alendronate.

### BRIEF DESCRIPTION OF DRAWINGS

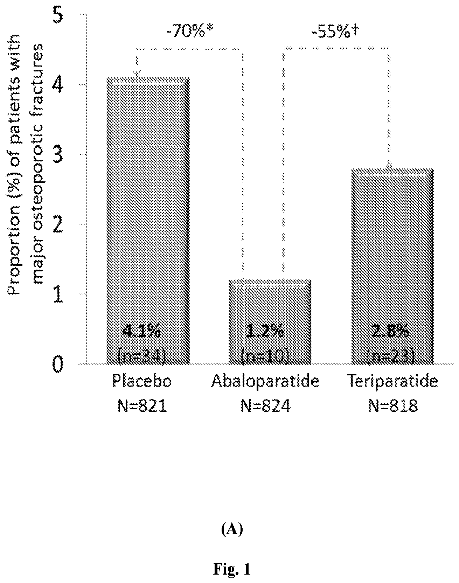

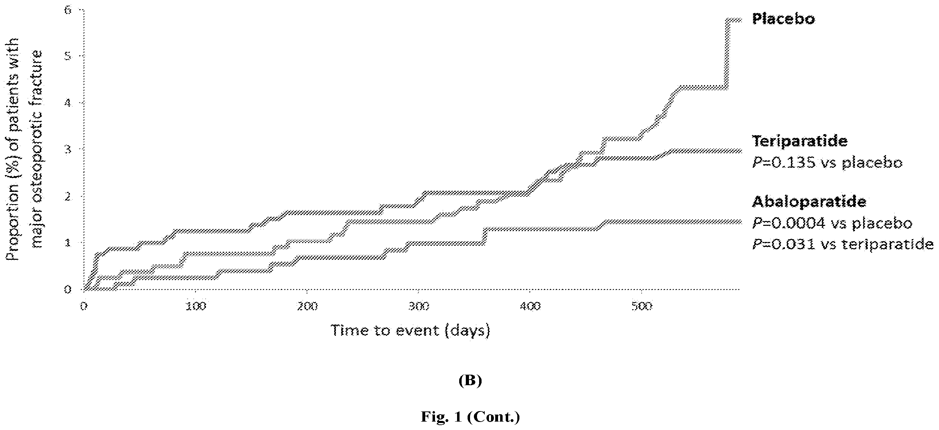

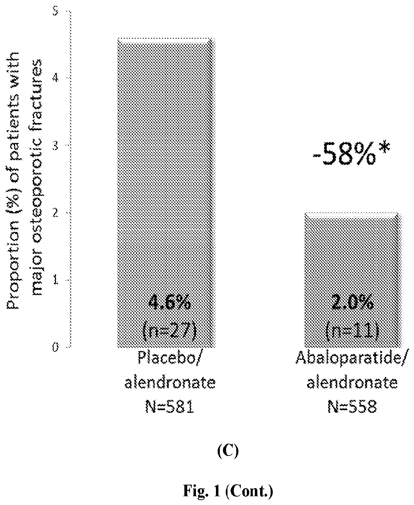

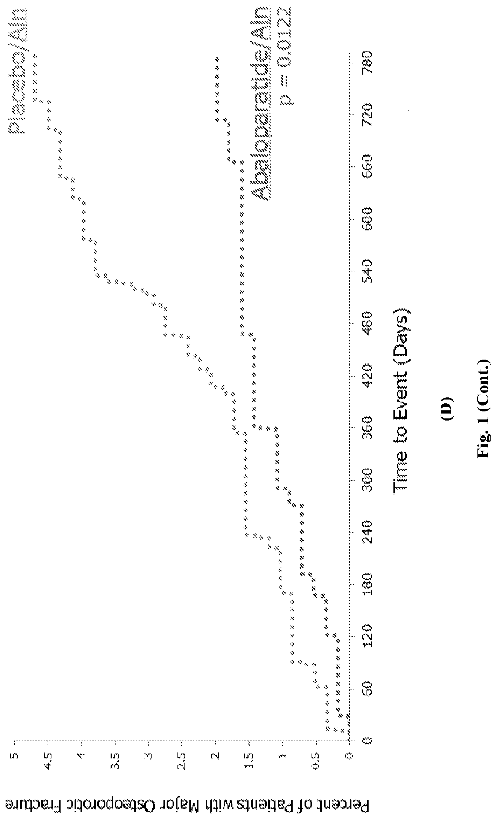

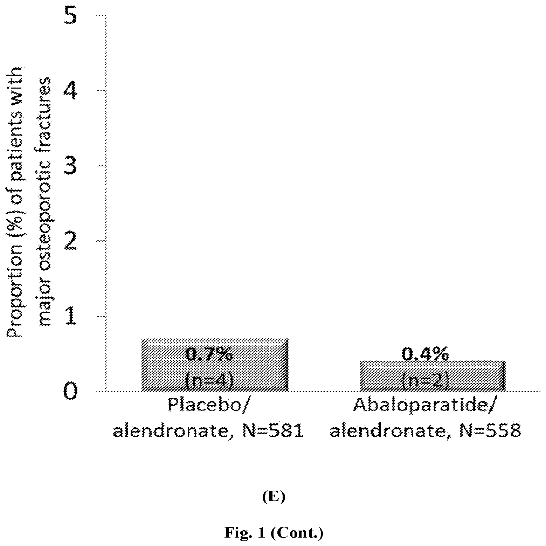

Figs. 1-4: Fractures in patient groups treated with placebo, abaloparatide, or teriparatide for 18 months. After a one-month follow-up visit after the 18 months of treatment, the placebo group and the abaloparatide group were subsequently treated with alendronate for another 6 months, which accounts for a total of 25 months of studies starting from the initiation of the treatment.
Fig. 1: Major osteoporotic fractures in all patient groups. A: Major osteoporotic fractures in all patient groups at the end of the 18-month treatment. B: Kaplan-Meier curve of major osteoporotic fractures in all patient groups during the 18-month treatments. C: Major osteoporotic fractures in patient groups treated with abaloparatide and alendronate or treated with placebo and alendronate at the end of the 25-month study. D: Kaplan-Meier curve of major osteoporotic fractures in patient groups treated with abaloparatide and alendronate or treated with placebo and alendronate during the 25-month study. E: Major osteoporotic fractures in patient groups treated with abaloparatide and alendronate or treated with placebo and alendronate during the 6-month treatments of alendronate.

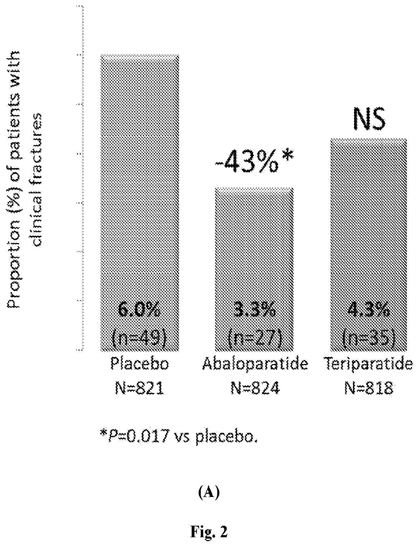

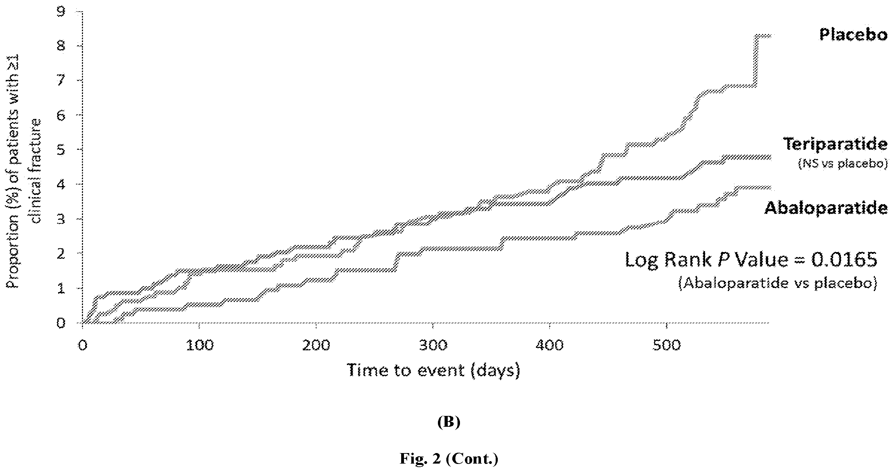

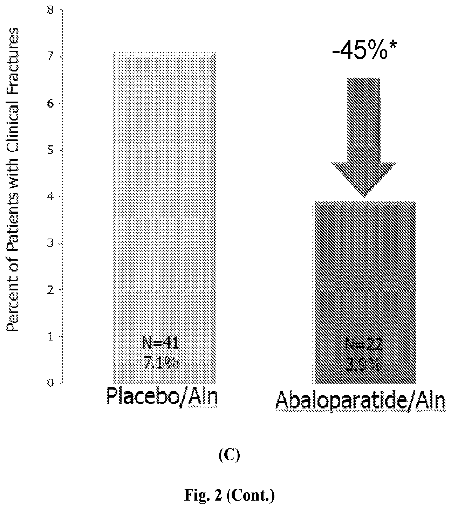

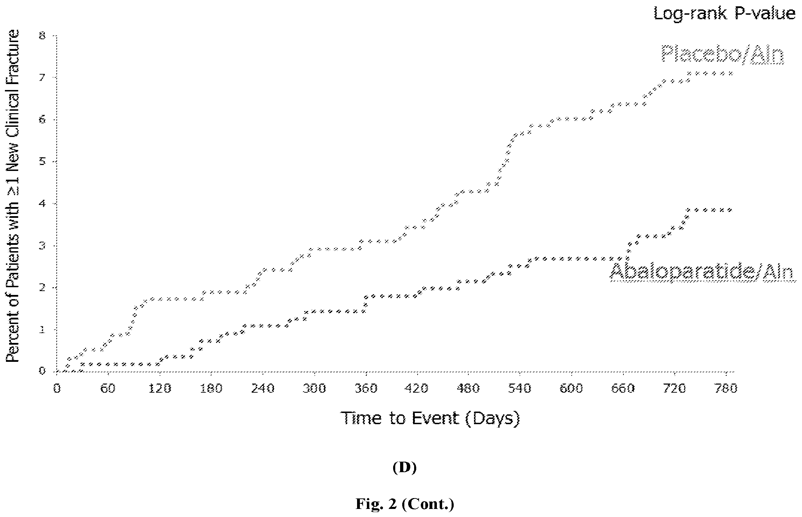

Fig. 2: Clinical osteoporotic fractures in all patient groups. A: Clinical osteoporotic fractures in all patient groups at the end of the 18-month treatment. B: Kaplan-Meier curve of clinical osteoporotic fractures in all patient groups during the 18-month treatments. C: Clinical osteoporotic fractures in patient groups treated with abaloparatide and alendronate or treated with placebo and alendronate at the end of the 25-month study. D: Kaplan-Meier curve of clinical osteoporotic fractures in patient groups treated with abaloparatide and alendronate or treated with placebo and alendronate during the 25-month study.

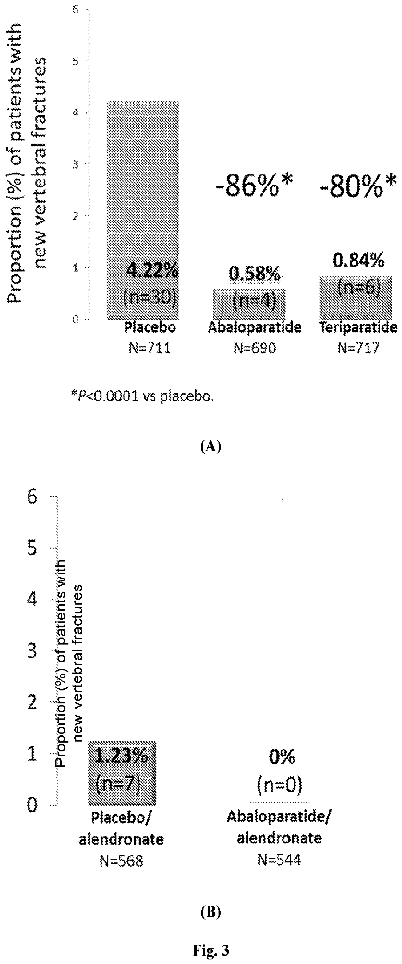

Fig. 3: New vertebral fractures in all patient groups. A: New vertebral fractures at the end of the 18-month treatments. B: New vertebral fractures in patient groups treated with abaloparatide and alendronate or treated with placebo and alendronate during the 6-month treatments of alendronate.

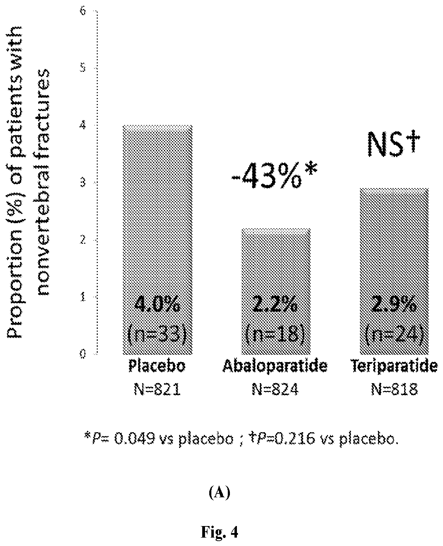

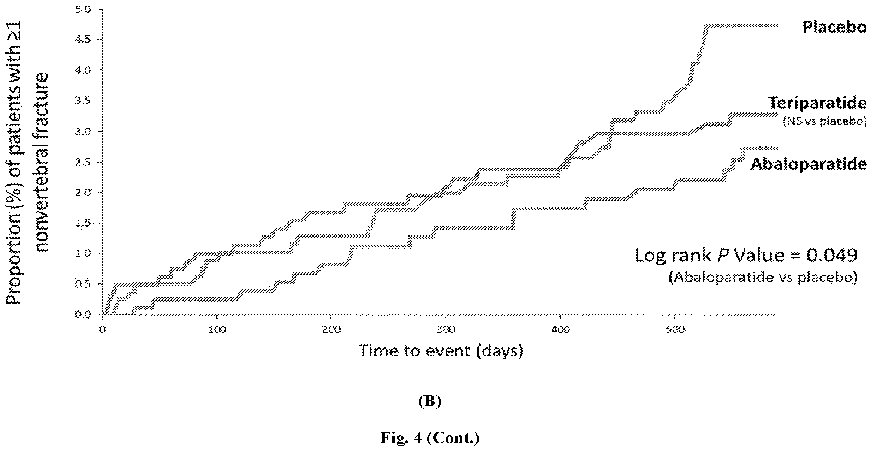

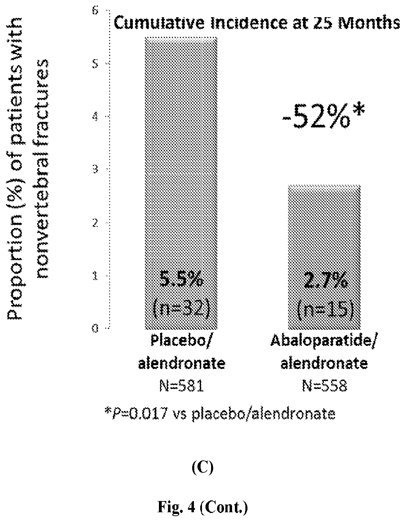

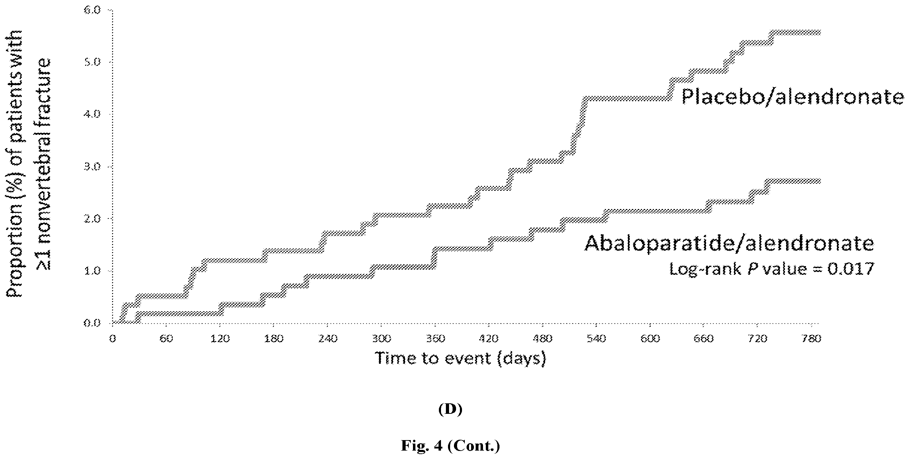

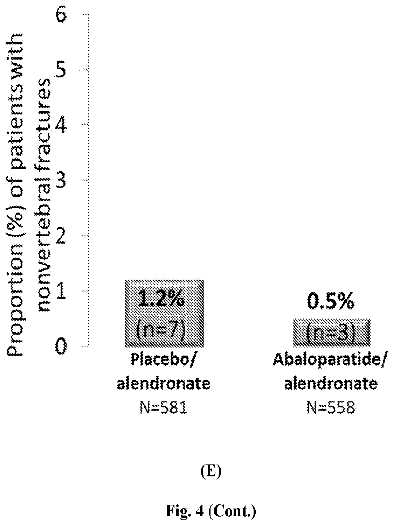

Fig. 4: Non-vertebral fractures in all patient groups. A: Non-vertebral fractures in all patient groups at the end of the 18-month treatment. B: Kaplan-Meier curve of non-vertebral fractures in all patient groups during the 18-month treatments. C: Non-vertebral fractures in patient groups treated with abaloparatide and alendronate or treated with placebo and alendronate at the end of the 25-month study. D: Kaplan-Meier curve of non-vertebral fractures in patient groups treated with abaloparatide and alendronate or treated with placebo and alendronate during the 25-month study. E: Non-vertebral fractures in patient groups treated with abaloparatide and alendronate or treated with placebo and alendronate during the 6-month treatments of alendronate.

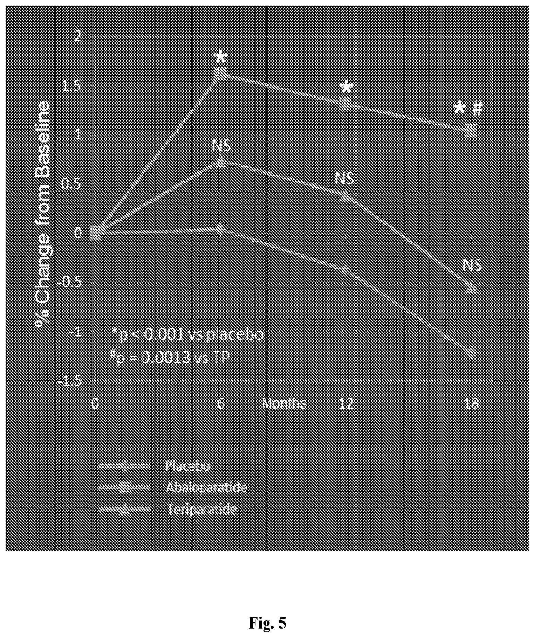

Fig. 5: Effect of abaloparatide to wrist BMD and wrist fracture reduction in all patient groups. (A): Changes in wrist BMD in all patient groups over 18 months: patients treated with placebo (diamond), patients treated with abaloparatide (square), and patients treated with teriparatide (triangle).

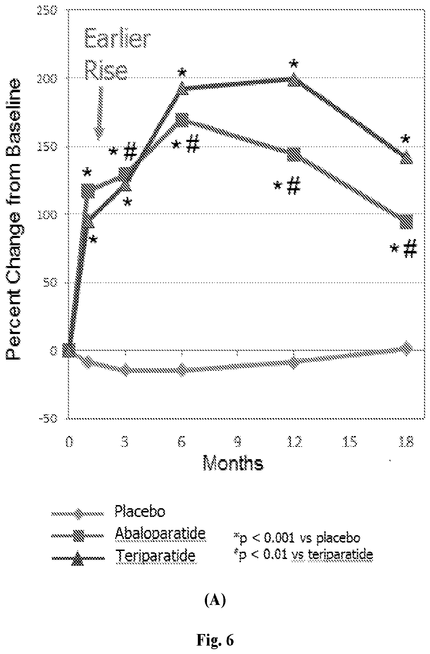

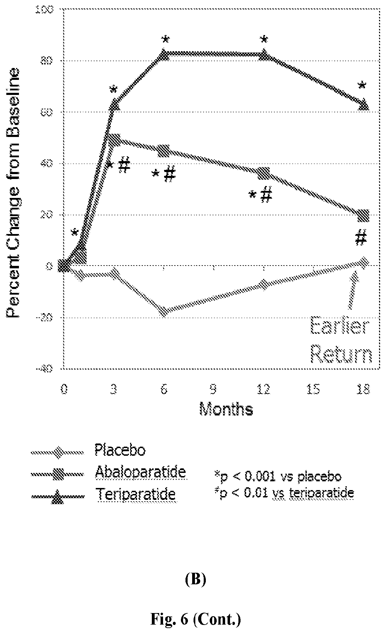

Fig. 6: Changes in bone turnover markers (CTX and P1NP) in all patient groups over 18 months: patients treated with placebo (diamond), patients treated with abaloparatide (square), and patients treated with teriparatide (triangle). A: Changes in P1NP in all patient groups. B: Changes in CTX in all patient groups. ^{∗}: *p*<0.001 vs. placebo. ^{#}: *p*<0.01 vs. teriparatide.

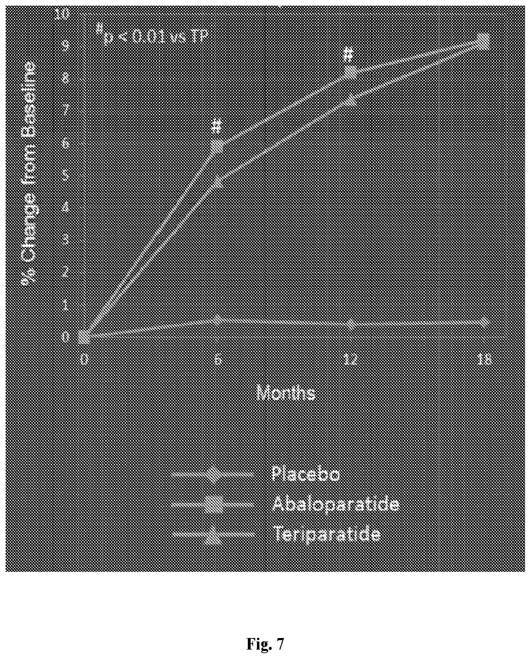

Fig. 7: Changes in BMD at the spine in all patient groups over 18 months: patients treated with placebo (diamond), patients treated with abaloparatide (square), and patients treated with teriparatide (triangle).

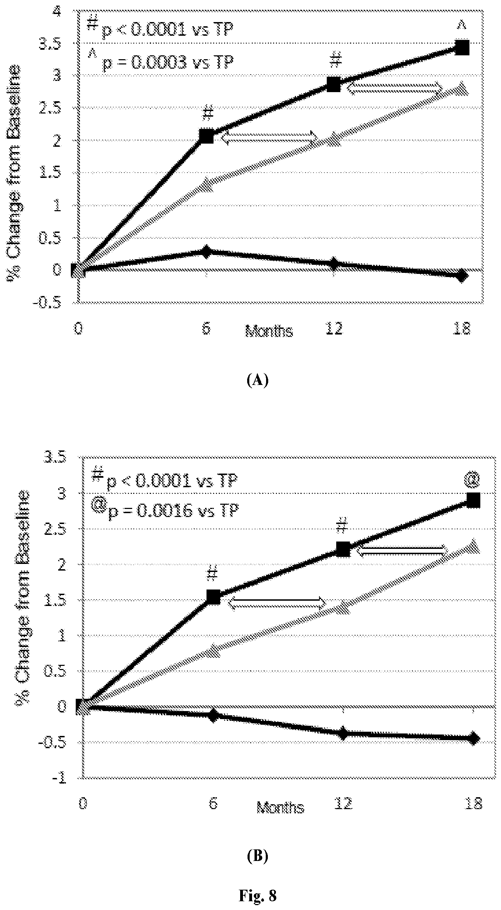

Fig. 8: Changes in BMD at non-vertebral sites in all patient groups over 18 months: patients treated with placebo (diamond), patients treated with abaloparatide (square), and patients treated with teriparatide (triangle). (A): Total hip BMD. (B): Femoral neck BMD.

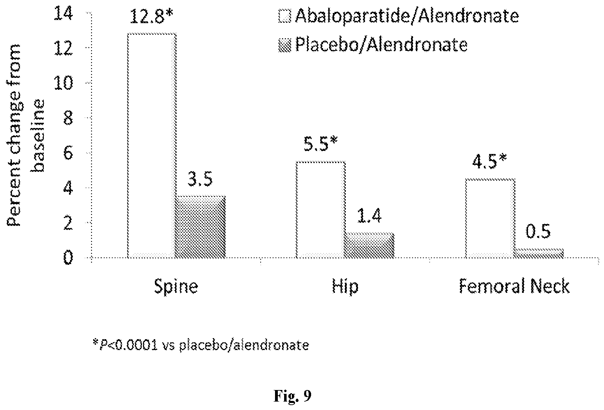

Fig. 9: Average BMD increase at month 25 following treatment with abaloparatide and alendronate (unfilled) or treatment with placebo and alendronate (filled) at spine, hip and femoral neck. The patents were treated with placebo or abaloparatide for 18 months, and subsequently treated with alendronate for another 6 months

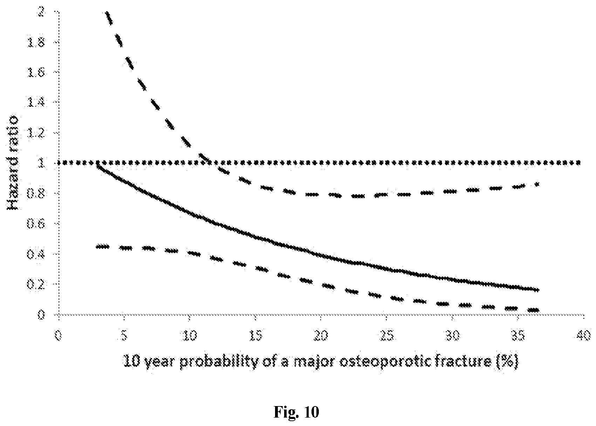

Fig. 10: Effect of abaloparatide on any clinical fracture compared to placebo, expressed as hazard ratio (HR), across the range of major osteoporotic fracture probabilities at baseline. The solid line represents the hazard ratio, while the dotted lines represent the variance/confidence interval for that hazard ratio.
Figs. 11-16: Unless otherwise specified, ABL represents abaloparatide, TPTD represents teriparatide, PBO represents placebo, and Veh represents vehicle.
Fig. 11: Subject Disposition for Example 3.
Fig. 12: Changes in BMD (mean percent change ±SE) at the spine in all patient groups over 24 weeks: patients treated with placebo (square), patients treated with abaloparatide at 20 µg (triangle), patients treated with abaloparatide at 40 µg (reversed triangle), patients treated with abaloparatide at 80 µg (diamond), and patients treated with teriparatide (filled circle). A: PA spine BMD. B: Femoral neck BMD. C: Total hip BMD. ^{∗}: p<0.01 versus placebo. %: p<0.05 versus placebo. &: *p*<0.05 versus teriparatide.
Fig. 13: Percentage of subjects who completed all study visits with a >3% increase in BMD after 24-weeks of treatment. ^{∗}: *p*<0.01 versus placebo. &: *p*<0.05 versus teriparatide and placebo.
Fig. 14: Changes in bone turnover markers (CTX, P1NP, and osteocalcin) in all patient groups over 24 weeks: patients treated with placebo (square), patients treated with abaloparatide at 20 µg (triangle), patients treated with abaloparatide at 40 µg (reversed triangle), patients treated with abaloparatide at 80 µg (diamond), and patients treated with teriparatide (filled circle). A: CTX. B: P1NP. C: Osteocalcin. a: p<0.002 versus placebo at 24 weeks. b: *p*<0.003 versus teriparatide at 24-weeks.
Fig. 15: Effect of abaloparatide treatment on BMD in ovariectomized (OVX) osteopenic rats. (A): BMD change from baseline at the lumbar spine. (B): Lumbar spine BMD. (C): BMD change from baseline at total femur. (D): Total femur BMD. (E): BMD change from baseline at cortical bone at the femoral shaft. (F): Femur midshaft BMD.
Fig. 16: Effect of abaloparatide treatment on trabecular bone microarchitecture in OVX rats. (A): Lumbar spine (L4). (B): Distal femur.

### DETAILED DESCRIPTION

The term "parathyroid hormone-related protein (PTHrP)" (not as claimed) refers to native human PTHrP (hPTHrP) and fragments thereof. The sequence of native hPTHrP (1-34) is: Ala Val Ser Glu His Gln Leu Leu His Asp Lys Gly Lys Ser Ile Gln Asp Leu Arg Arg Arg Phe Phe Leu His His Leu Ile Ala Glu Ile His Tur Ala (SEQ ID N0:2). PTHrP is a protein with homology to PTH at the amino-terminus that binds to the same G-protein coupled receptor. Despite a common receptor (PTHR), PTH primarily acts as an endocrine regulator of calcium homeostasis, whereas PTHrP plays a fundamental paracrine role in the mediation of endochondral bone development (11). The differential effects of these proteins may be related not only to differential tissue expression, but also to distinct receptor binding properties (12-14). Over the past several years, PTHrP has been investigated as a potential treatment for osteoporosis. The results of these studies have been mixed, with some suggesting that intermittent administration of high dose PTHrP increases bone formation without concomitant stimulation of bone resorption and others reporting measurable stimulation of bone resorption and significant hypercalcemia (15-17).

A "fragment" of hPTHrP (not as claimed) refers to a polypeptide having a sequence comprising less than the full complement of amino acids found in hPTHrP, which nonetheless elicits a similar biological response. Typically, fragments will be truncated from the C-terminus and will range from 30 to 40 residues in length.

As used herein (not as claimed), an "analogue" ofPTHrP refers to a polypeptide having between about 1 and about 20, between about 1 and about 15, or between about 1 and about 10 art- accepted substitutions, additions, or insertions relative to PTHrP (i.e., relative to hPTHrP or a fragment thereof), or combinations thereof, not to exceed atotal combination of 20 substitutions, additions, and insertions. As used herein (not as claimed), "insertions" include the insertion of an amino acid between two existing amino acids in the peptide chain. As used herein (not as claimed), "addition" means the addition of an amino acid to the Nor C terminus of the peptide chain. As used herein (not as claimed), "substitution" means the substitution of an amino acid for an existing amino acid in the peptide chain. As used herein (not as claimed), "art-accepted" substitutions, insertions, or additions are those which one of ordinary skill in the art would expect to maintain or increase the biological and/or hormonal activity of the peptide and not adversely affect the biological activity of the peptide. Art-accepted substitutions include, for example, substitution of one amino acid with a chemically or biologically similar amino acid, such as substituting one hydrophobic amino acid for another hydrophobic amino acid. PTHrP analogues are described with reference to their variation from the native sequence of hPTHrP.

The PTHrP analogue for use of the invention is abaloparatide. Abaloparatide was selected to retain potent anabolic activity with decreased bone resorption, less calciummobilizing potential, and improved room temperature stability (18). Studies performed in animals have demonstrated marked bone anabolic activity for the PTHrP analogue abaloparatide, with complete reversal of bone loss in ovariectomy-induced osteopenic rats and monkeys (19, 20).

As set forth in the Examples below (not as claimed), subjects treated with abaloparatide exhibited a significant reduction in certain bone fractures as compared to subjects treated with a placebo or with teriparatide.

When compared to subjects treated with placebo, subjects treated with abaloparatide showed a statistically significant reduction in major osteoporotic fractures, clinical fractures, new vertebral fractures, and non-vertebral fractures in an 18-month trial (see, e.g., Example 1, Table 1; not as claimed).

Subjects treated with teriparatide demonstrated a statistically significant reduction in new vertebral fractures compared to the placebo group (not as claimed). Compared to subjects treated with teriparatide, subjects treated with abaloparatide demonstrated a statistically significant reduction in major osteoporotic fractures (not as claimed).

Subjects treated with abaloparatide also showed a significant reduction in the risk of non-vertebral fractures (e.g., wrist fractures), and clinical fractures (see, e.g., Example 1, Table 1; not as claimed). Abaloparatide was further found to significantly decrease the risk of major osteoporotic fracture and any clinical fracture in postmenopausal women, irrespective of baseline fracture probability, using the Fracture Risk Assessment Tool **(FRAX).**

Subjects treated with abaloparatide exhibited a significant increase not only in BMD, but also in TBS (see, e.g., Example 4; not as claimed). TBS is a grey-scale textural analysis applied to spinal DXA images that has been shown to be correlated with trabecular bone microarchitecture and bone strength. TBS is also a predictor of fragility fractures of the spine and hip in postmenopausal women independent of BMD and other major clinical risk factors. As such, it captures additional patients at risk of fracture that are missed by BMD alone (35), and together with BMD more accurately captures bone strength.

Although a lower BMD is usually associated with higher fracture risk, a normal or even slightly higher than normal BMD does not necessarily indicate a lower fracture risk. For example, subjects with type II diabetes may have increased fracture risk (especially at the hips and/or wrists) despite a higher BMD (21). One factor behind the discrepancy between relatively normal **BMD** and high fracture risks may be the higher cortical porosity of subjects with diabetes (e.g., type II diabetes). For example, subjects with type II diabetes may have a cortical porosity up to twice that of controls (21). In certain embodiments, the abaloparatide and alendronate for use (in preventing or reducing non-vertebral bone fractures as more specifically defined herein) may be beneficial to subjects having diabetes and/or subjects having higher cortical porosity.

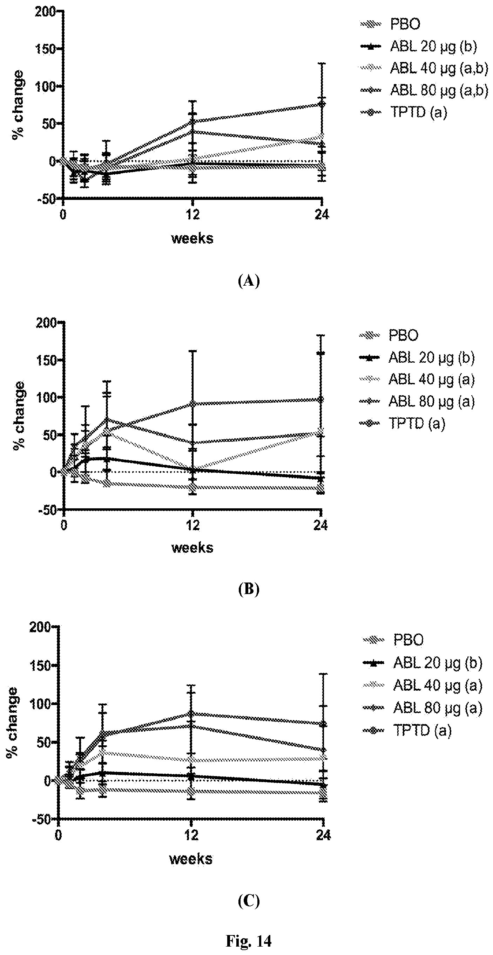

Subjects treated with abaloparatide for 18 months demonstrated significant **BMD** increase in total hip and femoral neck versus subjects treated with teriparatide (see, e.g., Example 1, Tables 4-5; not as claimed). Abaloparatide demonstrated a statistically significant increase in lumbar spine **BMD** at 6 months and 12 months versus teriparatide, and anon-statistically significant **BMD** increase at 18 months (see, e.g., Example 1, Tables 4-5; not as claimed). Without wishing to be bound by any theory, an earlier increase in bone formation marker P1NP in subjects treated with abaloparatide compared to subjects treated with teriparatide may contribute to the faster effects of abaloparatide on BMD (see, e.g., Example 1, Fig. 6A; and Example 3, Fig. 14B; not as claimed). For the CTX marker (bone resorption), subjects treated with abaloparatide showed an earlier return to the baseline at 18 months compared to subjects treated with teriparatide (see, e.g., Example 1, Fig. 6B; not as claimed).

Subjects treated with abaloparatide for 18 months followed by an alendronate for 6 months showed a significant reduction in fracture risk versus subjects treated with placebo for 18 months followed by similar alendronate therapy (see, e.g., Example 1, Table 2).

Exemplary non-vertebral bone fractures which may exhibit reduced fracture risk include, without limitation, wrist, hips, and clinical non-vertebral fractures (e.g., non-vertebral fractures with or without high trauma, confirmed through x-ray scan, radiologist report, emergency room/urgent care reports, hospital discharge reports, surgery reports, hospital or clinical notes, or other medical confirmation).

In certain embodiments, the alendronate and abaloparatide for use as claimed (preventing or reducing non-vertebral bone fractures) improve BMD and/or trabecular bone score TBS in the subject. Examples of bones which may exhibit improved BMD and/or TBS following administration include, without limitation, the total hip, wrist, femur, cortical bone of the femur (femoral diaphysis), and/or femoral neck in the subject. Exemplary non-vertebral bone fractures that may exhibit reduced fracture risk include, without limitation, major osteoporotic fracture, wrist, hip, clinical fracture.

The anti-resorptive therapeutic agent for use in the present invention is the bisphosphonate alendronate.

The term "subject in need thereof' as used herein refers to a mammalian subject, e.g., a human. In certain embodiments, a subject in need thereof has a fracture risk higher than normal. In certain embodiments, the abaloparatide and alendronate for use (as more specifically defined herein) are for use in a subject having low BMD and high cortical porosity. BMD may be measured by digital X-ray radiogrammetry (DXR) or other methods known in the art. As used herein, the term "low BMD" means a BMD T-score ≤ about 2 or ≤ about - 2.5, e.g., at one or more sites selected from the group consisting of spine (e.g., lumbar spine), hip (e.g., total hip or femoral neck), and wrist. As used herein, the term "cortical porosity" means the fraction of cortical bone volume that is not occupied by the bone. Cortical porosity may be measured by DXR or other methods known in the art to provide an estimation of the local intensity minima ("holes") in the cortical bone regions using a recursive (climbing) algorithm starting from the outer region (10). A combined porosity measure is derived from the area percentage of holes found in the cortical part relative to the entire cortical area, by averaging over the involved bones and scaling to reflect a volumetric ratio rather than the projected area. A "high cortical porosity" means a porosity of about 10% higher, about 15% higher, about 20% higher, about 50% higher, about 100% higher, or about 150% higher than that of healthy subjects from the same age group as controls. For example, the subject may have a cortical porosity of about 0.01256, which the control group has a cortical porosity of about 0.01093 (10). Subjects having a high cortical porosity may have a slightly low BMD, a normal BMD, or even a slightly higher than normal BMD, e.g., a BMD T-score of at least about -2, at least about -1.5, at least about -1, at least about -0.5, at least about -0.25, at least about -0.2, at least about -0.1, at least about 0, about -2 to about 3, about -2 to about 2.5, about -2 to about 2, about -2 to about 1.5, about -2 to about 1, about -2 to about 0.5, about -2 to about 0.25, about -2 to about 0.2, about -2 to about 0.1, or about -2 to about 0. For example, subjects with type II diabetes may have a cortical porosity up to twice that of controls while having normal or even slightly higher than normal BMD (21). Examples of suitable subjects in need thereof include, without limitation, women, women with osteoporosis and/or diabetes (e.g., type I or type II diabetes), postmenopausal women, postmenopausal women with osteoporosis and/or diabetes (e.g., type I or type II diabetes), and men with osteoporosis and/or diabetes (e.g., type I or type II diabetes).

The term "therapeutically effective amount" as used herein refers to an amount of a compound or agent that is sufficient to elicit the required or desired therapeutic and/or prophylactic response, as the particular treatment context may require. In certain embodiments, the therapeutically effective amount is an amount of the composition that yields maximum therapeutic effect. In other embodiments, the therapeutically effective amount yields a therapeutic effect that is less than the maximum therapeutic effect. For example, a therapeutically effective amount may be an amount that produces a therapeutic effect while avoiding one or more side effects associated with a dosage that yields maximum therapeutic effect. A therapeutically effective amount for a particular composition will vary based on a variety of factors, including but not limited to the characteristics of the therapeutic composition (e.g., activity, pharmacokinetics, pharmacodynamics, and bioavailability), the physiological condition of the subject (e.g., age, body weight, sex, disease type and stage, medical history, general physical condition, responsiveness to a given dosage, and other present medications), the nature of any pharmaceutically acceptable carriers in the composition, and the route of administration. One skilled in the clinical and pharmacological arts will be able to determine a therapeutically effective amount through routine experimentation, namely by monitoring a subject's response to administration of a composition and adjusting the dosage accordingly. For additional guidance, see, e.g., Remington: The Science and Practice of Pharmacy, 22nd Edition, Pharmaceutical Press, London, 2012, and Goodman & Gilman's The Pharmacological Basis of Therapeutics, 12th Edition, McGraw-Hill, New York, NY, 2011.

Examples of therapeutically effective amounts of abaloparatide include, without limitation, 10 µg to 250 µg, 50 µg to 200 µg, 50 µg to 150 µg, 70 µg to 100 µg, 70 µg to 90 µg, 75 µg to 85 µg, about 20 µg, about 40 µg, about 60 µg, about 80 µg, about 100 µg, about 120 µg, about 150 µg, about 200 µg, or about 250 µg. Other examples of therapeutically effective amounts of abaloparatide may also include, without limitation, about 5 µg/kg or about 20 µg/kg. One skilled in the art can select a therapeutically effective amount of the alendronate. The amount of the alendronatecan be further optimized subsequent to the therapy of abaloparatide.

Abaloparatide is administered by subcutaneous injection.

Abaloparatide is administered once a day for 18 months in the present invention.

In certain embodiments of the invention, the alendronate is administered once a day, twice a day, three times a day, or more than three times a day. In other embodiments, administration may occur once every 2 days, once every 3 days, once every 4 days, once per week, once per month. In certain embodiments, the alendronate is administered once a day.

In certain embodiments, the abaloparatide and alendronate for use provided herein reduce the wrist fracture risk of subjects treated with abaloparatide by 40% to 70%, 50% to 65%, 55% to 60%, or at least 58% when compared to untreated subjects or subjects treated with placebo. In certain embodiments, the wrist fracture risk for subjects treated with abaloparatide is reduced by 40% to 80%, 50% to 75%, 60% to 75%, 65% to 75%, 70% to 75%, or at least 72% compared to subjects treated with teriparatide.

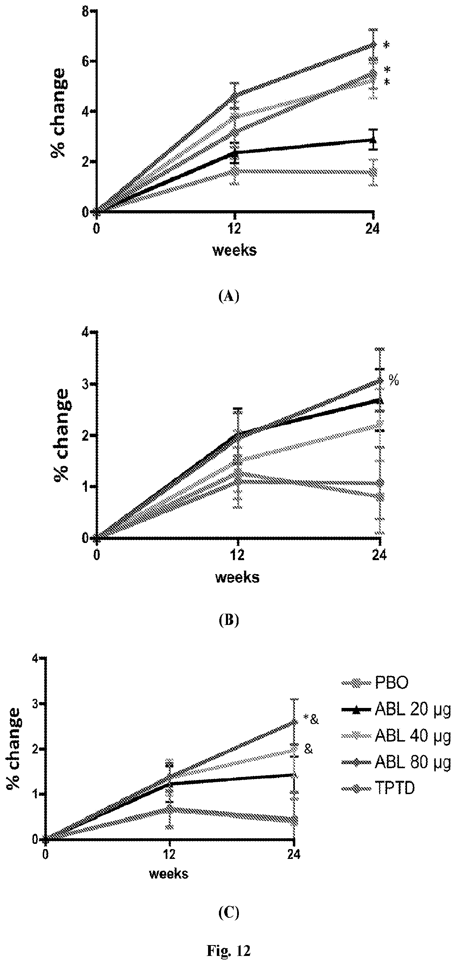

In certain embodiments of the invention, subjects are administered with abaloparatide at a daily dose of 20 µg, 40 µg, or 80 µg for 18 months and then administered with alendronate for 6 months with a dosage of 10 mg/day or 70 mg/week (e.g., oral), 5 mg/day or 35 mg/week (e.g., oral), 15 mg/day or 105 mg/week (e.g., oral), 20 mg/day or 140 mg/week (e.g., oral), 5 to 20 mg/day or 35 to 140 mg/week (e.g., oral), 5 to 15 mg/day or 35 to 105 mg/week (e.g., oral), 5 to 10 mg/day or 35 to 70 mg/week (e.g., oral), or 10 to 20 mg/day or 70 to 140 mg/week (e.g., oral). In certain embodiments of the invention, this results in a significant increase in BMD in the femoral neck and total hip (see, e.g., Fig. 12). In certain embodiments of the invention BMD at the femoral neck may increase by at least 2.2%, at least 2.7%, at least 3%, at least 3.1%, at least 4.5%, at least 5%, at least 6%, 1.5% to 4%, 2% to 4%, 2.5% to 4%, 2% to 3.5%, 1.5% to 6%, 2% to 6%, 2.5% to 6%, 1.5% to 5%, 2% to 5%, 2.5% to 5%, 1.5% to 4.5%, 2% to 4.5%, or 2.5% to 4.5%; and BMD for the total hip may increase by at least 1.4%, at least 2.0%, at least 2.6%, at least 3%, at least 3.5%, at least 4%, at least 4.5%, at least 5%, at least 5.5%, at least 6%, at least 7%, 0.6% to 3%, 1% to 3%, 1.5% to 3%, 0.6% to 3.5%, 1% to 3.5%, 1.5% to 3.5%, 0.6% to 4%, 1% to 4%, 1.5% to 4%, 2% to 4%, 0.6% to 4.5%, 1% to 4.5%, 1.5% to 4.5%, 2% to 4.5%, 0.6% to 5%, 1% to 5%, 1.5% to 5%, 2.0% to 5%, 0.6% to 5.5%, 1% to 5.5%, 1.5% to 5.5%, 2% to 5.5%, 0.6% to 6%, 1% to 6%, 1.5% to 6%, 2% to 6%, 0.6% to 6.5%, 1% to 6.5%, 1.5% to 6.5%, 2.0% to 6.5%, 0.6% to 7%, 1% to 7%, 1.5% to 7%, or 2% to 7%.

In certain methods disclosed herein (not as claimed), abaloparatide is administered in combination with one or more additional osteoporosis therapies, including for example an alendronate therapy. In these disclosures (not as claimed), the additional osteoporosis therapy may be administered before, during, or after the treatment with abaloparatide. Abaloparatide and the additional osteoporosis therapy may be administered separately or as part of the same composition. Administration of the two agents may occur at or around the same time, e.g., simultaneously, or the two agents may be administered at different times.

In certain embodiments, abaloparatide and alendronate are administered in pharmaceutical compositions as the active ingredient(s). Such pharmaceutical compositions may further comprise a pharmaceutically acceptable carrier. A "pharmaceutically acceptable carrier" as used herein refers to a pharmaceutically acceptable material, composition, or vehicle that is involved in carrying or transporting a compound or molecule of interest from one tissue, organ, or portion of the body to another tissue, organ, or portion of the body. A pharmaceutically acceptable carrier may comprise a variety of components, including but not limited to a liquid or solid filler, diluent, excipient, solvent, buffer, encapsulating material, surfactant, stabilizing agent, binder, or pigment, or some combination thereof. Each component of the carrier must be "pharmaceutically acceptable" in that it must be compatible with the other ingredients of the composition and must be suitable for contact with any tissue, organ, or portion of the body that it may encounter, meaning that it must not carry a risk of toxicity, irritation, allergic response, immunogenicity, or any other complication that excessively outweighs its therapeutic benefits.

Examples of pharmaceutically acceptable carriers that may be used in conjunction with the compositions described herein (not as claimed) include, but are not limited to, (1) sugars, such as lactose, glucose, sucrose, or mannitol; (2) starches, such as com starch and potato starch; (3) cellulose and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, com oil and soybean oil; (10) glycols such as propylene glycol; (11) polyols such as glycerin, sorbitol, mannitol and polyethylene glycol; (12) esters, such as ethyl oleate and ethyl laurate; (13) disintegrating agents such as agar or calcium carbonate; (14) buffering or pH adjusting agents such as magnesium hydroxide, aluminum hydroxide, sodium chloride, sodium lactate, calcium chloride, and phosphate buffer solutions; (15) alginic acid; (16) pyrogen-free water; (17) isotonic saline; (18) Ringer's solution; (19) alcohols such as ethyl alcohol and propane alcohol; (20) paraffin; (21) lubricants, such as talc, calcium stearate, magnesium stearate, solid polyethylene glycol, or sodium lauryl sulfate; (22) coloring agents or pigments; (23) glidants such as colloidal silicon dioxide, talc, and starch or tri-basic calcium phosphate; (24) other non-toxic compatible substances employed in pharmaceutical compositions such as acetone; and (25) combinations thereof.

In certain embodiments, abaloparatide is administered as a pharmaceutical composition having a pH range of 2 to 7, 4.5 to 5.6, or about 5.1.

### Examples

### Example 1. Evaluation of the PTHrP analogue abaloparatide for use in the reduction of fractures in postmenopausal women with osteoporosis.

The ACTIVE phase 3 fracture prevention trial was conducted for abaloparatide in postmenopausal women with osteoporosis who were otherwise healthy. The enrolled subjects were treated with 80 micrograms (µg) of abaloparatide, a matching placebo, or the approved daily dose of 20 µg of teriparatide for 18 months. The ACTIVE trial evaluated fracture rates, fracture risks, BMD, and bone turnover biomarkers (e.g., CTX and Pl NP) in all patient groups. Eligible subjects in the abaloparatide and placebo treatment groups continued in an extension study (ACTIVExtend), in which they received an approved alendronate therapy for osteoporosis management for 6 months and were evaluated for fracture incidence.

Fracture risk reduction and hazard ratio (HR) were derived from Kaplan-Meier (KM) curve. The abaloparatide treatment group exhibited a significant reduction in the risk of non-vertebral fractures (e.g., wrist) and clinical fractures (excluding fingers, toes, sternum, patella, skull and facial bones). When compared to placebo group, the abaloparatide treatment group showed a statistically significant reduction in major osteoporotic fractures, clinical fractures, new vertebral fractures and non-vertebral fractures both during the ACTIVE trial and the ACTIVExtend study (Tables 1 and 2). Compared to subjects treated with placebo, subjects treated with teriparatide demonstrated statistically significant fracture reduction only in new vertebral fractures, but did not show a statistically significant reduction in major osteoporotic fractures, clinical fractures, or non-vertebral fractures (Table 1).

Furthermore, abaloparatide demonstrated a statistically significant reduction in major osteoporotic fractures and wrist fractures versus teriparatide. In fact, the teriparatide group showed a fracture risk higher than that of the placebo group for wrist fractures.

**Table 1: Fracture Risk Reduction after 18-month ACTIVE Trial**

| Fig. No. | Fracture Type | Fracture Rate | | | Fracture Risk Reduction | | |
|---|---|---|---|---|---|---|---|
| | | PBO | ABL | TPTD | ABL v. PBO | TPTD v. PBO | ABL v. TPTD |
| 1A | Major osteoporotic fractures | 4.1% | 1.2% | 2.8% | 70% (p=0.0004) | 33% (p=0.135) | 55% (p=0.0309) |
| 2A | Incident clinical fractures | 6.0% | 3.3% | 4.3% | 43% (p=0.0165) | 29% (NS) | 19% (95% CI=0.43-1.45) |
| 4A | Incident non-vertebral fractures | 4.0% | 2.2% | 2.9% | 43% (*p*=0.0489) | 28% (p=0.2157) | 21% (NS) |
| | Wrist | 1.8% | 0.8% | 2.1% | 51% (*p*=0.1080) | -13% (*p*=0.7382) | 57% (*p*=0.0521) |

| | | | | | | | |
|---|---|---|---|---|---|---|---|
| NS: not statistically significant | | | | | | | |

**Table 2: Fracture Risk Reduction at Month 25 in ACTIVExtend Study**

| Fig. No. | Fracture | Fracture Rate | | Fracture Risk Reduction | P Value |
|---|---|---|---|---|---|
| | | PBO/ ALN | ABL/ ALN | PBO/ALN v. ABL/ALN | |
| 1C | Major osteoporotic fractures | 4.6% | 2.0% | 58% | 0.0122 |
| 2C | Incident clinical fractures | 7.1% | 3.9% | 45% | 0.0210 |
| 3B | New incident vertebral fractures | 4.4% | 0.55% | 87% | <0.0001 |
| 4C | Incident non-vertebral fractures | 5.5% | 2.7% | 52% | 0.0168 |

BMD and bone turnover biomarkers (CTX and P1NP) were also evaluated in all patient groups to compare the effects of abaloparatide versus teriparatide.

At all sites tested, including spine (e.g., lumbar spine), hip and femoral neck, patients treated with abaloparatide for 18 months followed by a treatment with alendronate for 6 months exhibited a significant BMD increase (Fig. 9). More patients in abaloparatide treatment group than in the placebo group achieved BMD threshold response as shown in Table 7.

Abaloparatide also demonstrated a statistically significant BMD increase versus teriparatide in total hip BMD and femoral neck BMD through the 18-month ACTIVE trial (Tables 4-5). Abaloparatide demonstrated a statistically significant BMD increase versus teriparatide in lumbar spine at 6 months and 12 months, and a non-statistically significant BMD increase at 18 months (Tables 4-5).

The abaloparatide group (square) demonstrated an earlier rise (at about one month) in P1NP marker (bone formation) compared to the teriparatide group (triangle) (Fig. 6A). For CTX marker (bone resorption), abaloparatide (square) showed an earlier return (at 18 months) compared to the teriparatide group (triangle) (Fig. 6B).

### Trial Design:

The ACTIVE pivotal Phase 3 fracture prevention trial for the PTHrP analogue abaloparatide, Study BA058-05-003 (see ClinicalTrials.gov), was a randomized, double-blind, placebo-controlled trial in postmenopausal osteoporotic women randomized to receive daily doses of one of the following for 18 months: 80 micrograms (µg) of abaloparatide; a matching placebo; or the approved daily dose of 20 µg of teriparatide. Treatment with abaloparatide at a daily dose of 80 µg or placebo remained blinded to all parties throughout the study. Teriparatide used was a proprietary prefilled drug and device combination that could not be repackaged. Therefore, its identity could not be blinded to treating physicians and patients once use began. Study medication was self-administered daily by subcutaneous injection for a maximum of 18 months. All enrolled patients also received calcium and vitamin D supplementation from the time of enrollment until the end of the treatment period. It was recommended to patients that they also continue these supplements through the one-month follow-up period.

The trial completed enrollment in March 2013 with 2,463 patients at 28 medical centers in 10 countries in the United States, Europe, Latin America, and Asia. The baseline characteristics of the selected patients are detailed in Table 3 below.

**Table 3: Baseline Characteristics of the Selected Patients for ACTIVE Studies**

| | Placebo (N=821) | Abaloparatide (N=824) | Teriparatide (N=818) | Overall (N=2,463) |
|---|---|---|---|---|
| Age (years) | 68.7 | 68.9 | 68.8 | 68.8 |
| Age groups (%) | | | | |
| < 65 years | 19.6 | 18.4 | 18.5 | 18.8 |
| 65 to 74 | 62.4 | 62.7 | 61.5 | 62.2 |
| > 74 | 18 | 18.8 | 20.0 | 19.0 |
| Baseline prevalent vertebral fracture (%) | 22.9 | 21.5 | 26.9 | 23.8 |
| Prior non-vertebral fracture history (%) | 50.7 | 49.2 | 45.4 | 48.4 |
| Lumbar spine (LS) BMD T-score | -2.9 | -2.9 | -2.8 | -2.9 |
| Total hip (TH) BMD T-score | -1.9 | -1.9 | -1.8 | -1.9 |
| Femoral neck (FN) BMD T-score | -2.2 | -2.2 | -2.1 | -2.1 |

The study enrolled otherwise healthy ambulatory women aged 49 to 86 (inclusive) who had been postmenopausal for at least five years, met the study entry criteria, and had provided written informed consent. The women enrolled in the study had a BMD T-score ≤ - 2.5 at the lumbar spine or hip (femoral neck) by dual-energy X-ray absorptiometry (DXA), and radiological evidence of two or more mild or one or more moderate lumbar or thoracic vertebral fractures, or history of low trauma forearm, humerus, sacral, pelvic, hip, femoral or tibial fracture within the past five years. Postmenopausal women older than 65 who met the above fracture criteria but had a T-score of <-2.0 could also be enrolled. Women at age 65 or older who did not meet the fracture criteria could also be enrolled if their T-score was <-3.0. All patients were to be in good general health as determined by medical history, physical examination (including vital signs), and clinical laboratory testing. This study population contained a patient population reflective of the type of severe osteoporosis patients that specialists would be expected to treat in their practices.

As set forth in the ACTIVE protocol, the primary efficacy endpoint was the number of patients treated with abaloparatide with incident vertebral fractures at the end of treatment as compared to those who received placebo. The pre-specified secondary efficacy parameters included, among other endpoints, reduction in the incidence/risk of non-vertebral fractures; changes in BMD of the spine, hip, and femoral neck from baseline to end of treatment as assessed by DXA and as compared to teriparatide; and the number of hypercalcemic events in abaloparatide treated patients when compared to teriparatide at end of treatment.

Safety evaluations performed in the ACTIVE trial included physical examinations, vital signs, 12-lead electrocardiograms, or ECGs, clinical laboratory tests and monitoring, and recording of adverse events. Specific safety assessments included pre-dose and post-dose (four hours) determination of serum calcium, determination of creatinine clearance, post-dose ECG assessments at selected visits, and assessments of postural hypotension (60 minutes post-dose) at selected clinic visits.

Each of the patients in abaloparatide 80 µg and placebo groups in the Phase 3 ACTIVE trial were eligible to continue in an extension study (ACTIVExtend), in which they are receiving an approved alendronate therapy for osteoporosis management. Key endpoints for the abaloparatide development program are the reduction in incident vertebral and non-vertebral fractures at up to 24 months in all randomized patients, including abaloparatide-treated and placebo-treated patients, all of whom are treated with alendronate in ACTIVExtend.

The ACTIVExtend study included an administration of alendronate (10 mg/day or 70 mg/week, oral) to the patients for 6 months following treatment with abaloparatide 80 µg/day for 18 months (N=558). The data was collected at month 25. The placebo group was also treated with alendronate for the same time period (N=581).

### Results

### Fracture Risk Reduction

On the secondary endpoints as compared to placebo, abaloparatide achieved a statistically significant fracture-risk reduction of 43% (*p*=0.0489, 95% CI=0.32-1.00) in the adjudicated non-vertebral fracture subset of patients (placebo group: n=33, fracture rate 4.0%; and abaloparatide group: n=18, fracture rate 2.2%)(Fig. 4A); a statistically significant reduction of 43% (*p*=0.0165, 95% CI=0.35-0.91) in the adjudicated clinical fracture group, which includes both vertebral and non-vertebral fractures (placebo group: n=49, fracture rate 6.0%; and abaloparatide group: n=27, fracture rate 3.3%) (Fig. 2A); and a statistically significant difference in the time to first incident non-vertebral fracture in both the adjudicated non-vertebral fracture (Fig. 4B) and the clinical fracture subset of patients (Fig. 2B). The open-label teriparatide [rDNA origin] injection treatment group, as compared to placebo, achieved a fracture-risk reduction of 28% (p=0.2157, 95% CI=0.42-1.22) in the adjudicated non-vertebral fracture subset of patients (Fig. 4A) and a reduction of 29% (95% CI=0.46-1.09) in the adjudicated clinical fracture group (Fig. 2A). The fracture-risk reduction observed in the abaloparatide treatment group, as compared to open-label teriparatide, was not statistically significant (Figs. 2A and 4A, and Table 1).

Alternatively, the primary endpoint of incident vertebral fracture reduction was performed excluding worsening vertebral fractures and including only new vertebral fractures (Figs. 3A and 3B). Using this analysis, on the primary endpoint of reduction of new vertebral fractures (excluding worsening), abaloparatide (N=690, n=4, fracture rate 0.58%) achieved a statistically significant 86% reduction as compared to the placebo-treated group (N=711, n=30, fracture rate 4.22%) (^{∗}: *p*<0.0001) (Fig. 3A). The open-label teriparatide injection treatment group (N=717, n = 6, fracture rate 0.84%) showed a statistically significant 80% reduction of new vertebral fractures (excluding worsening) as compared to the placebo-treated group (^{∗}: *p*<0.0001) (Fig. 3A).

As shown in Figs. 1A and 1B, after 18 months of treatment, abaloparatide unexpectedly demonstrated a significant reduction of 70% (95% CI=0.15-0.61) of the risk of major osteoporotic fractures as compared to placebo (Fig. 1A, ^{∗}: *p*=0.0004, abaloparatide v. placebo), and a significant reduction of 55% in the risk of major osteoporotic fractures as compared to teriparatide group (Fig. 1A, †: *p*=0.0309, abaloparatide v. teriparatide). However, risk of major osteoporotic fractures in group treated with teriparatide showed not statistically significant reduction of 33%compared to placebo (*p*=0.135, 95% CI=0.39-1.14). The risk of major osteoporotic fracture was reduced significantly more by abaloparatide than by teriparatide (**HR** *0.45,p*=*0.0309,* 95% CI=0.21-0.95). Abaloparatide also demonstrated significantly improved effects on major osteoporotic fractures as compared to teriparatide at 18 months. As shown in Figs. IC and ID, at 25th month patients (N=558) treated with abaloparatide for 18 months and followed by an alendronate treatment for another 6 months demonstrated significant reduction of 58% in the risk of major osteoporotic fractures as compared to placebo who were treated with alendronate only without the precedent treatment of abaloparatide (N=581) (p=0.0122). Fig. IE shows that during the six months of alendronate treatment, patients previously treated with abaloparatide for 18 months (N=558) had reduced risk of major osteoporotic fractures (n=2) as compared to placebo who were treated with alendronate only without the precedent treatment of abaloparatide (N=58 1, n=4).

As shown in Figs. 2A and 2B, at 18 moths abaloparatide demonstrated a significant reduction of 43% in the risk of clinical fractures as compared to placebo (p=0.0165). Abaloparatide also demonstrated improved effects on clinical fractures as compared to teriparatide at 18 months. As shown in Figs. 2C and 2D, at 25 months patients treated with abaloparatide for 18 months and followed by an alendronate treatment for another 6 months demonstrated significant reduction of 45% in the risk of clinical fractures as compared to placebo who were treated with alendronate only without the precedent treatment of abaloparatide (p=0.0210).

As shown in Figs. 3A and 3B, at 18 moths abaloparatide demonstrated a significant reduction of 86% in the incidence of new vertebral fractures as compared to placebo (p<0.0001). Abaloparatide also demonstrated improved effects on new vertebral fractures as compared to teriparatide (80% reduction) at 18 months (p<0.0001). Fig. 3B further demonstrates that no patients treated with abaloparatide had a vertebral fracture during the 6 months alendronate treatment period.

As shown in Figs. 4A and 4B, at 18 moths abaloparatide demonstrated a significant reduction of 43% in the risk of non-vertebral fractures as compared to placebo (p=0.0489). Teriparatide demonstrated a NS reduction (28%) in the risk of non-vertebral fractures as compared to placebo (p=0.2157). Abaloparatide also demonstrated improved effects on non-vertebral fractures as compared to teriparatide at 18 months. As shown in Figs. 4C and 4D, at 25 months patients treated with abaloparatide for 18 months and followed by an alendronate treatment for another 6 months (N=558) demonstrated significant reduction of 52% (p=0.0168) in the risk of non-vertebral fractures as compared to placebo who were treated with alendronate only without the precedent treatment of abaloparatide (N=58 1 ). Fig. 4E shows that during the six months of alendronate treatment, patients previously treated
with abaloparatide for 18 months (N=558) had reduced risk of non-vertebral fractures (n=3) as compared to placebo who were treated with alendronate only without the precedent treatment of abaloparatide (N=581, n=7).

### BMD and Bone Turnover Biomarkers

Fig. 5A demonstrated changes in wrist BMD in all patient groups: placebo (diamond), patients treated with abaloparatide (square), and patients treated with teriparatide (triangle). In comparison to teriparatide, abaloparatide unexpectedly showed significant improvement in BMD maintenance at the ultra-distal radius at 18 months.

Fig. 6A and Fig. 6B demonstrated the changes in bone turnover markers: CTX (bone resorption) and P1NP (bone formation) in all patient groups: placebo (diamond), patients treated with abaloparatide (square), and patients treated with teriparatide (triangle). Fig. 6A and Fig. 6B demonstrate that for P1NP marker (bone formation), abaloparatide (square) showed earlier rise in about one month comparing to teriparatide (triangle); and for CTX marker (bone resorption), abaloparatide (square) showed earlier return at 18 months comparing to teriparatide (triangle).

Comparative analyses of abaloparatide versus teriparatide were completed on the following BMD secondary endpoints using a Mixed-Effect Model for Repeated Measures (MMRM) method, shown in Table 4 below:

**Table 4: Mean Percent Change in Bone Mineral Density (BMD) From Baseline (MMRM)**

| | Lumbar Spine | | | Total Hip | | | Femoral Neck | | |
|---|---|---|---|---|---|---|---|---|---|
| | 6 mo | 12 mo | 18 mo | 6 mo | 12 mo | 18 mo | 6 mo | 12 mo | 18 mo |
| Placebo | 0.60% | 0.45% | 0.63% | 0.31% | 0.09% | -0.10% | -0.13% | -0.41% | -0.43% |
| Abaloparatide | 6.58%^{∗∗} | 9.77%^{∗∗} | 11.20%^{∗} | 2.32%^{∗∗} | 3.41%^{∗∗} | 4.18%^{∗∗} | 1.72%^{∗∗} | 2.65%^{∗∗} | 3.60%^{∗∗} |
| Teriparatide | 5.25%^{∗} | 8.28%^{∗} | 10.49%^{∗} | 1.44%^{∗} | 2.29%^{∗} | 3.26%^{∗} | 0.87%^{∗} | 1.54%^{∗} | 2.66%^{∗} |

| | | | | | | | | | |
|---|---|---|---|---|---|---|---|---|---|
| ^{∗∗} *p*<0.0001 vs placebo and teriparatide ^{∗} *p*<0.0001 vs placebo | | | | | | | | | |

Comparative analyses of the PTHrP analogues abaloparatide and teriparatide were completed on the following BMD secondary endpoints using an ANCOVA approach, shown in Table 5 below:

**Table 5: Mean Percent Change In Bone Mineral Density (BMD) From Baseline (ANCOVA)**

| | Lumbar Spine | | | Total Hip | | | Femoral Neck | | |
|---|---|---|---|---|---|---|---|---|---|
| | 6 mo | 12 mo | 18 mo | 6 mo | 12 mo | 18 mo | 6 mo | 12 mo | 18 mo |
| Placebo | 0.55% | 0.39% | 0.48% | 0.29% | 0.10% | -0.08% | -0.12% | -0.37% | -0.44% |
| Abaloparatide | 5.90%^{∗∗} | 8.19%^{∗∗∗} | 920%^{∗} | 2.07%^{∗∗} | 2.87%^{∗∗} | 3.44%^{∗∗∗∗} | 1.54%^{∗∗} | 2.21%^{∗∗} | 2.90%^{∗∗∗∗∗} |
| Teriparatide | 4.84%^{∗} | 7.40%^{∗} | 9.12%^{∗} | 1.33%^{∗} | 2.03%^{∗} | 2.81%^{∗} | 0.80%^{∗} | 1.41%^{∗} | 2.26%^{∗} |

| | | | | | | | | | |
|---|---|---|---|---|---|---|---|---|---|
| ^{∗} vs. placebo *p*<0.0001 ^{∗∗} vs. teriparatide *p*<0.0001 ^{∗∗∗} vs. placebo *p*<0.0001 AND vs. teriparatide *p*=0.0087 ^{∗∗∗∗} vs. placebo *p*<0.0001 AND vs. teriparatidep=0.0003 ^{∗∗∗∗∗} vs. placebo *p*<0.0001 AND vs. teriparatide *p*=0.0016 | | | | | | | | | |

Bone resorption: Changes in bone resorption showed a significant difference between patients treated with abaloparatide and patients treated with teriparatide. At all timepoints, CTX increased significantly more in the teriparatide group than in the group treated with abaloparatide. While abaloparatide showed a transient elevated level of CTX compared to placebo, teriparatide showed a persistent elevated level of CTX compared to placebo. The difference in CTX levels between abaloparatide group and teriparatide group may indicate different "anabolic windows" between the two treatments. At 18 months, the CTX level in the group treated with abaloparatide was statistically insignificant compared to placebo; whereas teriparatide showed elevated levels compared to placebo.

Bone formation: Changes in bone turn-over showed a different pattern from changes in bone resorption. The P1NP level of the teriparatide group was higher than that of the group treated with abaloparatide while the difference of the P1NP levels was not so significant as the difference in the CTX levels. The P1NP levels of both treatment groups were significantly higher than that of the placebo at all time points.

Fig. 7 demonstrates changes in BMD at the spine in all patient groups: placebo (diamond), patients treated with abaloparatide (square), and patients treated with teriparatide (triangle). Abaloparatide showed significantly greater BMD increase as compared to teriparatide at 6 and 12 months at lumbar spine.

Fig. 8 demonstrates changes in BMD at non-vertebral sites (total hip and femoral neck) in all patient groups: placebo (diamond), patients treated with abaloparatide (square), and patients treated with teriparatide (triangle). At all timepoints, abaloparatide and teriparatide showed significantly greater BMD increase as compared to placebo. Abaloparatide showed significantly greater BMD increase as compared to teriparatide at 6, 12, and 18 months at total hip and femoral neck. Moreover, there was a delay of about 6 months in the teriparatide group comparing to the group treated with abaloparatide to achieve the same level of BMD increase at total hip and femoral neck. Therefore, abaloparatide achieved significant results in rapid BMD response.

At month 6, 19.1% of subjects treated with abaloparatide showed increased BMD of >3% at all three sites (lumbar spine, total hip, femoral neck) compared to 0.9% for the placebo group and 6.5% for the teriparatide group. At 12 months, 33.2% of abaloparatide treated group had BMD increases of >3% compared to the placebo group (1.5%) or the teriparatide group (19.8%). At 18 months, 44.5% of abaloparatide treated group had BMD increases of >3% compared to the placebo group (1.9%) or the teriparatide group (32.0%). All of the differences were statistically significant, *p*<0.0001

Fig. 9 demonstrates that at all sites tested, including spine (e.g., lumbar spine), hip and femoral neck, the patients treated with abaloparatide for 18 months followed by a treatment with alendronate for 6 months exhibited a significant BMD increase.

Additionally, Table 6 demonstrates the percentage of patients with BMD increase at the spine, hip and femoral neck at 25 months. More patients in abaloparatide treatment group achieved BMD threshold response.

**Table 6: Percentage of Patients with BMD Increase at the Spine. Hip and Femoral Neck**

| BMD | Placebo (%) | Abaloparatide (%) | P Value |
|---|---|---|---|
| > 0% | 40.0 | 83.1 | < 0.0001 |
| > 3% | 7.4 | 51.7 | < 0.0001 |
| > 6% | 0.5 | 20.4 | < 0.0001 |

### Efficacy:

Fig. 4B demonstrates the Kaplan-Meier curve of time to first incident non-vertebral fractures by treatment group in the intent-to-treat population (excluding fingers, toes, sternum, patella, skull and facial bones). Fig. 2B demonstrates the Kaplan-Meier curve of time to first incident clinical fractures by treatment group in the intent-to-treat population (excluding fingers, toes, sternum, patella, skull and facial bones). The Kaplan-Meier curves show a significant reduction in the risk of non-vertebral and clinical fractures in the group treated with abaloparatide.

### Safety:

The ACTIVE trial also evaluated several potential safety measures, including blood calcium levels, orthostatic hypotension, nausea, dizziness, and injection-site reactions. The adverse events (AEs) reported by ≥5% in any treatment group were summarized below in Table 7 for groups treated with placebo, abaloparatide, and teriparatide, respectively.

**Table 7: AE Reported for Patient Groups (N = 2460)**

| Most Frequently Reported AEs reported by ≥5% in any treatment group | Placebo, n=820 | Abaloparatide, n=822 | Teriparatide, n=818 |
|---|---|---|---|
| Hypercalcemia^{∗} | 0.37% | 3.41%^{†} | 6.37%^{†} |
| Hypercalciuria | 9.0% | 11.3% | 12.5%^{‡} |
| Dizziness | 6.1% | 10.0%^{‡} | 7.3% |
| Arthralgia | 9.8% | 8.6% | 8.6% |
| Back Pain | 10.0% | 8.5% | 7.2%^{‡} |
| Nausea | 3.0% | 8.3%^{‡} | 5.1%^{‡} |
| Upper respiratory tract infection | 7.7% | 8.3% | 8.9% |
| Headache | 6.0% | 7.5% | 6.2% |
| Hypertension | 6.6% | 7.2% | 5.0% |
| Influenza | 4.8% | 6.3% | 4.2% |
| Nasopharyngitis | 8.0% | 5.8% | 6.5% |
| Urinary tract infection | 4.6% | 5.2% | 5.0% |
| Palpitations | 0.4% | 5.1%^{‡} | 1.6%^{‡} |
| Pain in extremity | 6.0% | 4.9% | 5.1% |
| Constipation | 5.1% | 4.5% | 4.2% |

| | | | |
|---|---|---|---|
| ^{∗}Serum albumin-corrected calcium value ≥10.7 mg/dL; †*p*=0.006 abaloparatide vs teriparatide; ‡*p*<0.05 vs placebo. | | | |

Each of the abaloparatide group and teriparatide group had statistically significantly higher hypercalcemia event rates as compared to the placebo group, and the abaloparatide group had a statistically significant lower hypercalcemia event rate as compared to the teriparatide group (p=0.006).

The safety measures were also performed in a population of 1133 patients treated with alendronate during the ACTIVExtend study. The adverse events of patients treated with alendronate are detailed in Table 8 below. Abaloparatide showed a favorable safety profile, was well tolerated.

**Table 8: Adverse Events of Patients Treated with Alendronate**

| Most Frequently Reported AEs (N = 1133) | Placebo/Alendronate (n = 580) | Abaloparatide/Alendronate (n = 553) |
|---|---|---|
| Arthralgia | 4.7% | 4.3% |
| Dyspepsia | 2.2% | 2.7% |
| Upper Respiratory Tract Infection | 4.5% | 2.5% |
| Urinary Tract Infection | 1.0% | 2.4% |
| Bone Pain | 1.2% | 2.2% |
| Diarrhea | 1.4% | 2.0% |
| Hypercalciuria | 1.6% | 2.0% |
| Influenza | 1.0% | 2.0% |
| Nasopharyngitis | 1.4% | 2.0% |
| Abdominal pain, upper | 2.6% | 1.8% |
| Back pain | 2.1% | 1.6% |
| Pain in extremity | 2.4% | 1.3% |
| Hypertension | 2.1% | 1.1% |

### Comparative Example 2. Efficacy of the PTHrP analogues abaloparatide for prevention of major osteoporotic fracture or any fracture.

This example demonstrates the efficacy of the PTHrP analogue abaloparatide versus baseline fracture risk using the FRAX tool.

Fracture risk assessment, and FRAX specifically, is well known in the art (see, e.g., Unnanuntana et al., "Current Concepts Review: The Assessment of Fracture Risk," J Bone Joint Surg Am. 92: 743-753 (2010)). Briefly, FRAX is a prediction tool for assessing an individual's risk of fracture by incorporating non-BMD clinical risk factors, including age, sex, weight, height, previous fracture, parent fractured hip, current smoking, alcohol, or glucocorticoids, rheumatoid arthritis, and secondary osteoporosis, in addition to or in alternative to femoral neck BMD. FRAX can estimate a country-specific 10-year probability of hip fracture and a 10-year probability of a maj or osteoporotic fracture (clinical spine, forearm, hip or shoulder fracture).

Baseline clinical risk factors (such as age, BMI, prior fracture, glucocorticoid use, rheumatoid arthritis, smoking and maternal history of hip fracture) were entered into country- specific FRAX models to calculate the 10-year probability of major osteoporotic fractures with or without inclusion of femoral neck BMD. The interaction between probability of a major osteoporotic fracture and treatment efficacy was examined by a Poisson regression.

821 women randomized to the placebo group and 824 women in abaloparatide were followed for up to 2 years. At baseline, the 10-year probability of major osteoporotic fractures (with BMD) ranged from 2.3-57.5%. Treatment with abaloparatide was associated with a 69% decrease in major osteoporotic fracture (MOF) compared to placebo treatment
(95% CI: 38-85%). The risk of any clinical fracture (AF) decreased by 43%; (95% CI: 9-64%). Hazard ratios for the effect of abaloparatide on the fracture outcome did not change significantly with increasing fracture probability (p>0.30 for MOF and p=0.11 for AF (Fig. 10)). Similar results were noted for the interaction when FRAX probability was computed without inclusion ofBMD.

Therefore, abaloparatide significantly decreased the risk of major osteoporotic fracture and any clinical fracture in postmenopausal women, irrespective of baseline fracture probability.

### Comparative Example 3. Effects of the PTHrP analogue abaloparatide on BMD at the lumbar spine, total hip, and femoral neck in postmenopausal women with osteoporosis.

### Patients and methods

### Study Subjects

Healthy postmenopausal women between the ages of 55 to 85 (based on a 5-year history of amenorrhea and an elevated serum level of FSH) were enrolled in the study if they met one of the following the following definitions of osteoporosis:
1) DXA-derived BMD T-score -2.5 at the lumbar spine or femoral neck or total hip.
2) DXA-derived BMD T-score -2.0 with a history of a prior low trauma forearm, humerus, vertebral, sacral, pelvic, hip, femoral, or tibial fracture within the past five years.
3) DXA-derived BMD T-score -2.0 with an additional osteoporosis risk factor such as age 65 years or strong maternal history of osteoporosis (defined as a fracture related to osteoporosis or osteoporosis itself determined by BMD criteria).

Women were required to have a body mass index (BMI) between 18.5 and 33 kg/m², normal levels of serum calcium, PTH (1-84), 25-hydroxy vitamin D, phosphorus, and alkaline phosphatase, and normal cardiovascular parameters (normal ECG, systolic blood pressure 100 and 155 mmHg, diastolic blood pressure 40 and 95 mmHg).

Women were excluded for a history of osteosarcoma or other bone disorders (e.g. Paget's disease or osteomalacia), radiation therapy, malabsorption, nephrolithiasis, urolithiasis, renal dysfunction (serum creatinine >1.5 mg/dL), or any medical condition that could interfere with the conduct of the study. Women with spine abnormalities that would prohibit assessment of **BMD** and those who had undergone bilateral hip replacement were also excluded. In terms of medications, subjects were excluded if they had been treated with calcitonin, estrogens, estrogen derivatives, selective estrogen receptor modulators, tibolone, progestins, anabolic steroids or daily glucocorticoids in the past six months, if they had received bisphosphonates or strontium in the past five years, or if they had ever received parathyroid hormone or its analogues, fluoride, gallium nitrate or denosumab.

### Study Design

This study (clinicaltrial.gov #NCT00542425) was a randomized, parallel-group, multi-center, dose-finding, double-blind placebo-controlled trial conducted at 30 study centers in the United States, Argentina, India, and the United Kingdom. All subjects provided informed written consent prior to initiating any study procedures. Subjects were screened for eligibility and then randomized to one of the following 24-week self-administered treatment groups: placebo subcutaneous injection daily, the PTHrP analogue abaloparatide (20-µg, 40-µg or 80-µg) subcutaneous injection daily, or teriparatide (Forteo^{®}; Eli Lilly) 20 µg subcutaneous injection daily. All subjects received supplemental calcium (500-1000 mg) and vitamin D (400-800 IU) per local practice. Patients and investigators remained blinded to treatment with abaloparatide and placebo throughout the study, although patients randomized to teriparatide were unblinded due to the need to use the marketed drug and delivery device. BMD was assessed by DXA at baseline and again 3 and 6 months after treatment initiation. Biochemical markers of bone turnover, serum abaloparatide levels, and anti-abaloparatide antibody formation measurements were obtained throughout the treatment period. Blood calcium levels were assessed 4-hours and 24-hours after drug administration. Subjects were monitored for adverse events (AEs) and local tolerance at the injection site at each visit. Clinical and laboratory safety parameters, electrocardiograms, were also measured at each study visit.

### Measurements

Dual X-ray absorptiometry: DXA scans were obtained at each local site and then sent to a central imaging reader (BioClinica Inc. Newton, PA) where they underwent a quality control review and then analyzed according to each manufacturers guidelines. Scans performed during the treatment period on the same instrument used for the baseline scan were acquired. Each study site performed Instrument Quality Control over time (instrument standardization and phantom calibration) that was reviewed by the central reader.

Biochemical Markers of bone turnover: Fasting morning blood samples (collected 24 hours after last injection if taking teriparatide) were obtained at each visit. Serum osteocalcin (OC) was measured via electrochemiluminescence assay (Roche Diagnostics, Basel, Switzerland), with intra-assay with coefficients of variation (CVs) of 1.8% and 4.8% respectively. Serum amino-terminal propeptide of type 1 procollagen (P1NP) was measured via radioimmunoassay (Orion Diagnostica, Espoo, Finland) with inter- and intra-assay CVs of 4.5% and 5.5% respectively. Serum β-c-terminal telopeptide of type one collagen (CTX) was measured via electrochemiluminescence assay (Roche Diagnostics, Basel, Switzerland) with inter- and intraassay CVs of 3.8% and 6.9% respectively.

### Statistical Analysis

Efficacy and safety were assessed using all randomized patients who received at least one dose of study drug. Baseline characteristics and safety parameters were summarized using descriptive statistics. The primary efficacy endpoints were changes from baseline to 24 weeks in BMD and bone turnover markers. The efficacy endpoints were analyzed using a mixed model repeated-measures analysis of the change at each visit, which included treatment group, study visit and treatment-by-visit interaction as the fixed effects. The variance-covariance matrix between visits was assumed to be unstructured. Comparisons of mean change from baseline for each abaloparatide dose versus placebo at Week 24 were assessed using this model in a sequential fashion, starting from the 80mg group, then the 40mg and lastly the 20mg. The comparison of teriparatide vs. placebo was done using this model as well. Due to the skewedness of percentage change from baseline in bone marker results, median and interquartile ranges are reported. For treatment comparisons, bone marker results were log transformed prior to performing the mixed model repeated-measures analysis. The dose response relationship of increasing doses of abaloparatide to increased efficacy response was assessed by testing a linear contrast of among the three abaloparatide dose groups and the placebo group using the same model but excluding the teriparatide group. In a post-hoc analysis, we also assessed the number (%) of patients who achieved a >3% BMD at the spine, femoral neck, total hip after 24-weeks of treatment in the placebo, teriparatide, and abaloparatide 80-µg groups only. The 3% threshold was chosen based on DXA scanner precision of approximately 1% corresponding to the least significant change (LSC) in BMD at the 95% confidence limits of 3% and to conform with prior responder analyses (22-28). In the responder analysis, only those patients who had both baseline and Week 24 BMD measurements were included (valid-completers). The difference in the number (%) of responders between treatment groups was assessed by the Chi-square test. All hypotheses were tested at the 2-sided 5% significance level. Because this was a Phase-II, dose-response, hypothesis generating study, p-values were not adjusted for multiple comparisons. The SAS System Version 8.2 (SAS Institute Inc.) was used for the statistical analysis.

### Extension Study

A 24-week extension was added as an amendment to the protocol while the study was underway. To be eligible for the extension, study subjects were requited to have been within two weeks of receiving their last treatment dose. A total of 69 patients were eligible for the extension and of those, 55 continued treatment to 48 weeks (placebo group n=11, abaloparatide 20-µg n=13, abaloparatide 40-µg n=10, abaloparatide 80-µg n=7, teriparatide 20-µg n=14). BMD was re-measured at the 48-week visit.

### Results

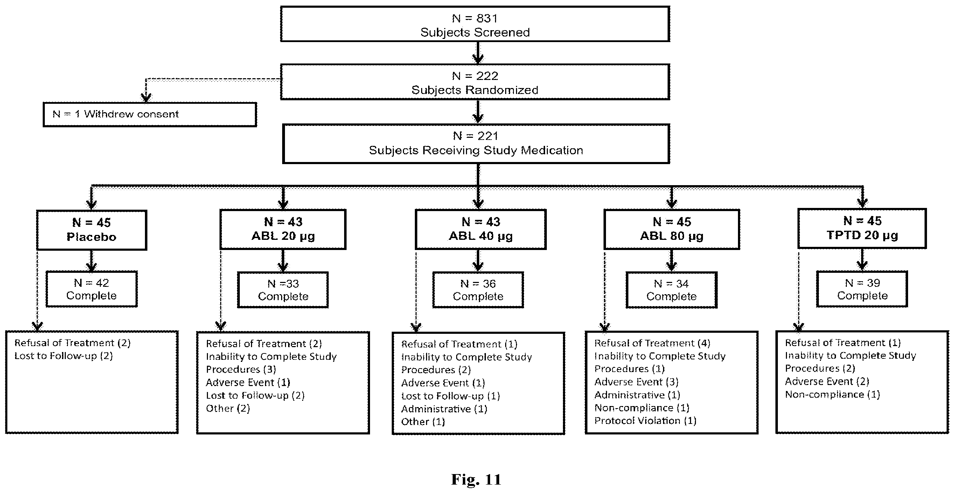

Fig. 11 shows the disposition of the study subjects. Of the 222 patients randomized, all but 1 received at least 1 dose of study drug, 191 (86%) patients had BMD measurements at 12 weeks, and 184 (83%) completed the study through the 24-week visit. Subjects in the 5 treatment groups were similar in regard to demographic and clinical characteristics, including baseline BMD measurements and levels of biochemical markers of bone turnover.

### Bone Mineral Density

Fig. 12 shows the 24-week changes in BMD of lumbar spine (Fig. 12A), femoral neck (Fig. 12B), and total hip (Fig. 12C) in the various treatment groups: patients treated with placebo (square), patients treated with abaloparatide at 20 µg (triangle), patients treated with abaloparatide at 40 µg (reversed triangle), patients treated with abaloparatide at 80 µg (diamond), and patients treated with teriparatide (filled circle).

Lumbar spine BMD: At 24-weeks, lumbar spine BMD (±SD) increased by 1.6 ±3.4% in the placebo group, 5.5 ±4.1% in the teriparatide group, and 2.9 ±2.6%, 5.2 ±4.5%, and 6.7 ±4.2% in abaloparatide 20, 40 and 80-µg groups, respectively. Compared to placebo, the increases in BMD in the 40 and 80-µg abaloparatide groups and the teriparatide group were statistically significant (*p*<0.001). The difference in the BMD increase between the abaloparatide 80-µg group and the teriparatide group was not statistically significant. Additionally, the effects of abaloparatide on lumbar spine BMD showed a significant dose response (linear trend) (*p*<0.001).

Femoral neck BMD: At 24-weeks, BMD at the femoral neck increased by 0.8 ±4.8% in the placebo group, 1.1 ±4.6% in the teriparatide group, and 2.7 ±4.0%, 2.2 ±4.4% and 3.1 ±4.2% in abaloparatide 20, 40 and 80-µg groups, respectively. Compared to placebo, the increases in femoral neck BMD in the 80-µg group was statistically significantly (p=0.036) whereas there were no significant differences in BMD increases between placebo-treated subjects and those treated with either teriparatide, abaloparatide 20-µg, or abaloparatide 40-µg. The difference between the increase in femoral neck BMD in the abaloparatide 80-µg group and the teriparatide group was not statistically significant (*p*=0.066).

Total Hip BMD: At 24-weeks, total hip BMD increased by 0.4 ±3.1% in the placebo group, 0.5 ±3.9% in the teriparatide group, and 1.4 ±2.6%, 2.0 ±3.7%, and 2.6 ±3.5% in abaloparatide 20, 40 and 80-µg groups, respectively. Compared to placebo, total hip BMD increased more in the abaloparatide 80-µg group only (p=0.007). Moreover, the BMD increase at the total hip was significantly greater in both the abaloparatide 40-µg and the abaloparatide 80-µg groups than in the teriparatide group (*p*=0.047 and p=0.006, respectively).

### Response to Therapy

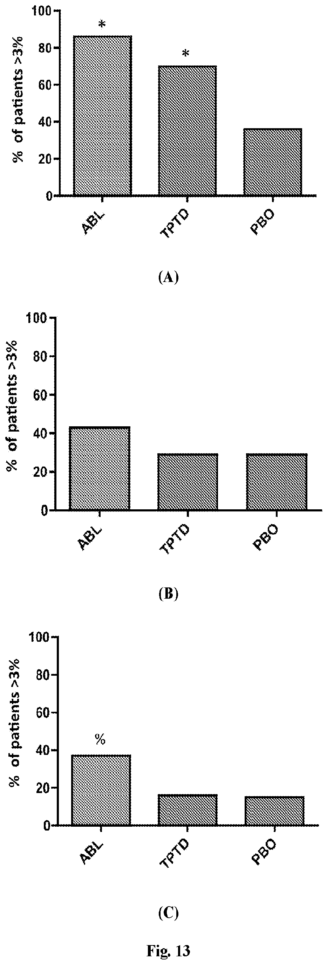

The results of the responder analyses are shown in Fig. 13. The percentage of subjects with a >3% BMD gain at the lumbar spine was higher in the abaloparatide group (80µg dose, 86%) than the placebo group (36%) (^{∗}*p*<0.001) but not the teriparatide group (70%) (p=0.092) (Fig. 13A). Furthermore, more abaloparatide-treated women had a >3% total hip BMD gain (37%) than those treated with teriparatide (16%, *p*<0.02) or placebo (15%, *p*<0.04) (Fig. 13C). There was no statistically significant difference in the percent of women experiencing >3% BMD increases at the femoral neck in any of the three groups (Fig. 13B).

### Biochemical Markers of Bone Turnover

Fig. 14 shows the 24-week changes in serum biochemical markers of bone formation (P1NP (Fig. 14B), OC (Fig. 14C)) and bone resorption (CTX, Fig. 14A) in the various treatment groups: patients treated with placebo (square), patients treated with abaloparatide at 20 µg (triangle), patients treated with abaloparatide at 40 µg (reversed triangle), patients treated with abaloparatide at 80 µg (diamond), and patients treated with teriparatide (filled circle). a: p<0.002 versus placebo at 24 weeks. b: *p*<0.003 versus teriparatide at 24-weeks

Bone formation: In the 40-µg and 80-µg abaloparatide groups (and the teriparatide group) P1NP began to increase by week 1. After 24-weeks, the median (interquartile range) of P1NP had increased by 55 (-2, 160)% in the 40-µg abaloparatide group, 52 (0, 158)% in the 80-µg abaloparatide group, and by 98 (21, 184)% in the teriparatide group (all changes statistically significantly different than placebo, which decreased by 20 (7, 28)%, *p*<0.001). P1NP increased more in the teriparatide group than in the 20-µg abaloparatide group (*p*<0.001) but the increase was not significantly different when compared to the two higher dose groups of abaloparatide. The pattern of the change in OC was generally similar to those observed in P1NP. For both markers, the effects of abaloparatide showed a significant dose response (linear trend) (*p*<0.001).

Bone resorption: Changes in bone resorption showed a slightly different pattern than those in bone formation with increases not apparent until week 12. After 24-weeks, the median (interquartile range) of CTX had increased by 32 (-13, 77)% in the 40-µg abaloparatide group, 23 (-9, 86)% in the 80-µg abaloparatide group, and by 76 (13, 130)% in the teriparatide group (all changes statistically significantly different than placebo, which decreased by 7 (-19, 26)%). CTX increased more in the teriparatide group than in any abaloparatide group (p<0.003). In contrast to markers of bone formation, there was no incremental increase in CTX between the 40-µg abaloparatide and 80-µg abaloparatide groups.

### Safety

During the 24-week treatment period, treatment-emergent AEs (TEAEs) were reported in 164 (74%) of 221 patients. The proportion of patients that experienced TEAEs was similar across treatment groups, with 71%, 72%, 74%, 76% and 78% in the placebo, abaloparatide 20,40 and 80 µg, and teriparatide groups, respectively. TEAEs considered by the investigator to be possibly or probably related to study treatment were reported in 66 (30%) of 221 patients, with 27%, 21%, 35%, 38% and 29% in placebo, abaloparatide 20, 40 and 80 µg, and teriparatide groups, respectively. The incidence of headache was numerically higher with abaloparatide 40-µg and 80-µg compared to placebo, with 7%, 5%, 14% and 13% of patients in the placebo, abaloparatide 20, 40 and 80-µg groups, respectively, and similar to teriparatide (13%). Dizziness was also highest with abaloparatide 80-µg, with 4%, 0%, 9%, 11% and 4% in the placebo, abaloparatide 20, 40 and 80-µg, and teriparatide groups, respectively. The majority of injection site reactions were of mild or moderate intensity and similar in the abaloparatide and teriparatide treatment groups. The majority of TEAEs were mild to moderate in severity. Eight patients (4%) experienced at least 1 event that was severe in intensity during 24-week study period; the incidence of severe events was similar across the treatment groups. Severe events included back and chest pain (placebo group), influenza, ascites and ovarian epithelial cancer (abaloparatide 20-µg group, diagnosed after 14 days of treatment), headache (abaloparatide 40-µg group), dyspepsia, syncope, diarrhea and upper abdominal pain (abaloparatide 80-µg group), and arthralgia and joint injury (teriparatide group). One event of severe intensity, syncope in a patient in the abaloparatide 80-µg group was assessed as probably related to study treatment; the event was reported as resolved within 1 day and did not require treatment. All other events of severe intensity were reported as unrelated to study treatment. Serious TEAEs were reported in three patients (1%): acute bronchitis in a placebo treated patient, ovarian cancer with ascites in a patient assigned to abaloparatide 20-µg and diverticulitis in a patient in the abaloparatide 80-µg group. None was categorized as treatment-related, and no deaths were reported. Seven patients (3%) discontinued due to AEs, including one each (2%) in the abaloparatide 20-µg and 40-µg groups, three patients (7%) in the abaloparatide 80-µg group and two patients (4%) in the teriparatide group. No clinically meaningful differences were noted between the placebo and active treatment groups for ECG parameters.

### Hypercalcemia

Serum calcium levels ≥10.5 mg/dL were observed 4-hours post-dose in 1 patient (2%) in the placebo group, 3 patients (7%) in the abaloparatide 20-µg group, 6 patients (14%) in the abaloparatide 40-µg, 5 patients (11%) in the abaloparatide 80-µg group, and 18 patients (40%) in the teriparatide group. The incidence of hypercalcemia at 4-hours was greater in the teriparatide group than in each abaloparatide group (*p*<0.01). When measured 24-hours after the last injection, serum calcium levels ≥10.5 mg/dL were observed in 1 patient (2%) in the placebo group, 2 patients (5%) in the abaloparatide 20-µg group, 3 patients (7%) in the abaloparatide 40-µg, 4 patients (9%) in the abaloparatide 80-µg group, and 7 patients (16%) in the teriparatide group (no significant between-group differences). The highest value obtained by any subject 4-hours post-dose were 10.5, 11.0, 11.2, 11.6, and 12.6 mg/dL in the placebo, abaloparatide 20-µg, abaloparatide 40-µg, abaloparatide 80-µg, and teriparatide groups, respectively. The highest value obtained by any patient 24-hours post-dose were 10.7, 11.3, 11.1, 10.7, and 11.2 mg/dL in the placebo, abaloparatide 20-µg, abaloparatide 40-µg, abaloparatide 80-µg, and teriparatide groups, respectively.

### Antibody Formation

After 24 weeks, 16 (12%) patients who had received abaloparatide demonstrated positive, low (≤1:20) anti-abaloparatide antibody titer. The number and types of AEs in this group were similar to AEs overall. No immune-related events were reported in antibody positive patients. One antibody-positive patient in the abaloparatide 40-µg group had evidence of in vitro abaloparatide neutralizing activity at 24 weeks, although there was no apparent evidence of efficacy attenuation in this patient (9.3% increase in total analyzable spine BMD at 24-weeks), or related safety events.

### Extension Study

The baseline demographic and baseline characteristics in the extension population were similar to those of the entire study cohort and the number of subjects per treatment group ranged from 7-14 women. At 48-weeks, lumbar spine BMD increased by 0.7%, 5.1%, 9.8%, 12.9%, and 8.6% in the placebo, abaloparatide 20, 40 and 80-µg groups, and the teriparatide group, respectively. Total hip BMD increased by 0.7%, 1.9%, 2.1%, 2.7%, and 1.3% in the placebo, abaloparatide 20, 40 and 80-µg groups, and the teriparatide group, respectively. Femoral neck BMD increased by 1.0%, 3.9%, 1.8%, 4.1%, and 2.2% in the placebo, abaloparatide 20, 40 and 80-µg groups, and the teriparatide group, respectively. Given the small numbers in the extension study, there were no significant between-group differences with the exception of spine BMD, which increased more in the abaloparatide 40-µg, abaloparatide 80-µg, and teriparatide groups as compared to placebo.

As in the entire cohort, tolerability was similar in all groups with treatment-related TEAEs occurring in 36%, 31%, 30%, 29% and 21% in the placebo, abaloparatide 20 µg, 40 µg, and 80 µg, and teriparatide groups, respectively. The most common AEs were arthralgia and urinary tract infection (each 15%), bronchitis, influenza and nasopharyngitis (each 9%), and anemia, back pain, dizziness, dyslipidemia, hypercalciuria, and injection site hematoma (each 7%). One SAE, joint swelling, was reported in a patient who received placebo and one SAE, hospitalization for repair of bilateral femoral hernia that was unrelated to treatment, was reported with abaloparatide 80-µg. One patient in the abaloparatide 40-µg group discontinued due to moderate syncope that was classified by the investigator as possibly related to abaloparatide.

### Discussion

In this study, 24-weeks of abaloparatide increased BMD in lumbar spine, femoral neck, and total hip. The magnitude of these increases were robust when compared to currently-available therapies. In the lumbar spine, a dose response relationship between abaloparatide at the tested doses and increases in BMD was shown. Moreover, at the hip, 40-µg and 80-µg daily dose of abaloparatide increased BMD more than the currently marketed 20-µg daily dose of teriparatide. Additionally, fewer women receiving 80-µg/day of abaloparatide lost BMD at the femoral neck and hip than those receiving teriparatide 20-µg daily. Finally, the BMD changes observed in the limited population enrolled in the extension study suggest that the BMD increased with abaloparatide remained relatively linear during the first year of treatment.

The physiological mechanisms underlying the distinct BMD effects observed with abaloparatide 80-µg versus teriparatide 20-µg are not clear. While both bone formation and bone resorption were stimulated by abaloparatide treatment, the magnitude of these increases (even at the higher doses tested) was lower than with teriparatide. Notably, the 24-week increase in bone formation markers was approximately 50% greater in the teriparatide group than in the abaloparatide 80-µg group whereas the increase in the resorption marker (CTX) was 100% higher. Thus, it is possible that the higher formation-to-resorption ratio in abaloparatide-treated women was a contributing factor to the differential effects of these two agents on BMD. Moreover, prior studies have suggested that the early effects of PTH and teriparatide at cortical sites such as the hip and radius are due to increased intracortical bone remodeling, leading to increased cortical porosity (29-32). Since the increase in the rate of bone resorption following the PTHrP analogue abaloparatide treatment was more limited and delayed compared to PTH, it is possible that earlier gains in BMD at sites with a higher proportion of cortical bone were also the result of an absolute lower rate of intracortical resorption hence less cortical porosity. It should be noted that the increase in cortical porosity at cortical bone-rich anatomic sites in teriparatide-treated patients was not associated with reduced estimated bone strength, an observation that may be due to improvement in trabecular microarchitecture (29-33). It remains to be tested whether the abaloparatide-induced increases in hip BMD, along with increases in trabecular bone as evidenced by the large spine BMD increases, will be associated with larger increases in estimated bone strength. Studies, assessing cortical and trabecular microarchitecture by in vivo imaging or bone biopsy may be useful in better defining the effects of abaloparatide on bone quality.

The molecular mechanisms underlying the differences between teriparatide and abaloparatide are unknown, but may relate to differing affinities of the two drugs to the specific conformations of the PTHR, as has been shown with PTH and PTHrP (12-14). Specifically, it has been reported that PTHrP activity at the PTHR is restricted to the cell surface, whereas teriparatide remains associated with the PTHR and it coupled G-protein and moves to internalized compartments of the cell, potentially acting as a persistent and active ternary complex. It is not yet clear if these differential receptor interactions account for the differences between PTH and PTHrP when used pharmacologically, or if the effects of abaloparatide are also impacted by distinct post-PTHR binding physiology.

The incidence of AEs were similar among groups, and most events were mild or moderate in intensity. Although a positive anti-abaloparatide antibody titer with low titers (≤1:20) was reported in 16 patients with abaloparatide, no immune-related events were reported. Of the five patients in the 80-µg daily dose group who developed antibodies in the first 24 weeks of exposure, all but one had an antibody titer of 1:1, and none were newly positive in the extension phase. Also notable was relatively low incidence of hypercalcemia observed in abaloparatide-treated subjects. This may be due to the lower rates of bone resorption observed in abaloparatide patients but differential effects in the kidney cannot be excluded.

In summary, 24-weeks of abaloparatide, especially at the 80-µg daily subcutaneous dose, increased BMD of the spine and hip in a potentially clinically meaningful way. The abaloparatide-induced increases in lumbar spine BMD were robust and the BMD increases at the total hip were greater than both placebo and teriparatide, as were the patient responserates at the hip and femoral neck. This capacity to increase BMD, along with the safety data presented, the low incidence ofhypercalcemia, and the room-temperature stability of the PTHrP analogue abaloparatide, support the continued investigation of abaloparatide as promising anabolic treatment for postmenopausal osteoporosis.

### Comparative Example 4. Effects of the PTHrP analogue abaloparatide on trabecular bone score (TBS) at the lumbar spine, total hip, and femoral neck in postmenopausal women with osteoporosis.

To assess the effects of the PTHrP analogue abaloparatide on trabecular microarchitecture as indirectly assessed by TBS, the TBS (TBS Calculator v2.2, Medimaps group, Plan-les-Ouates, Geneva, Switzerland) in a blinded fashion at 0, 12, and 24-weeks in 222 postmenopausal osteoporotic women (age 55-85) who were randomized to receive 24-weeks of daily subcutaneous injections of placebo, abaloparatide 20-µg, abaloparatide 40-µg. abaloparatide 80-µg, or teriparatide (TPTD) 20-µg was retrospectively calculated. Between groups differences in the mean percent TBS changes were assessed by unpaired t-test. Results:

Out of 221 women treated, 77 women could not be assessed as the DXA scanner was not compatible with TBS software. Subjects (N= 145) in the 5 treatment groups were similar in regard to demographic and clinical characteristics, including baseline BMD measurements and levels of biochemical markers of bone turnover. After 12-weeks, TBS increased significantly by+ 1.2%, +1.7%, +1.9% and+1.5% in the abaloparatide 20-µg, abaloparatide 40-µg, abaloparatide 80-µg and TPTD groups, respectively and decreased by - 0.2% in the placebo group (PBO). The 12-week mean percent increases in TBS in the abaloparatide 40-µg and abaloparatide 80-µg treatment groups were significantly greater than in the placebo group (bothp=0.05). After 24-weeks, TBS increased by +2.4%, +2.7%, +3.6% and +2.6% in the abaloparatide 20-µg, abaloparatide 40-µg, abaloparatide 80-µg and TPTD groups, and decreased by -1.1% in the placebo group (PBO). The 24-week increases in TBS were significantly greater in all treatment groups compared to the change in the placebo group (p<0.005).

### Summary:

24-weeks of treatment with abaloparatide significantly improved trabecular microarchitecture as indirectly assessed by TBS. Combined with the effects of abaloparatide on BMD, these results support the further investigation of abaloparatide as an anabolic therapy in postmenopausal osteoporosis.

### Comparative Example 5. Effects of the PTHrP analogues abaloparatide on vertebral and femoral BMD, microarchitecture and strength in ovariectomized (OVX) osteopenic rats.

The bone anabolic effect of six weeks daily administration of the PTHrP analogue abaloparatide to adult ovariectomized **(OVX)** osteopenic rats were assessed. Bone mass in **OVX** osteopenic rats received marked gains in response to abaloparatide treatment. Gains in bone mass were observed not only in the trabecular bone compartment of the lumbar spine and the femur, but also at the cortical bone of the femur (femoral diaphysis). These dose depended gains in bone mass were associated with improved bone microarchitecture and increased bone biomechanical properties.

### Materials and Methods

### Animals

All procedures, protocols and study designs were reviewed, approved and overseen by the Institutional Care and Use Committee (IACUC) at Radius Health. 10 week old female Sprague-Dawley rats (Charles River Laboratories) were housed individually in ventilated, polycarbonate cages with access to food and water ad libitum. Their environment was maintained at 18-26°C with 30-70% relative humidity and a 12 hour light/dark cycle.

### Experimental design

Sprague-Dawley rats were either sham-operated (Sham) or ovariectomized (OVX) at 12 weeks of age and remained untreated for 8 weeks (bone depletion period). Osteopenic OVX rats (n = 20-24/group) were treated once daily by subcutaneous injection (SC) with vehicle (0.9% NaCl), abaloparatide 5 µg/kg or abaloparatide 20 µg/kg for 6 weeks. Sham rats were treated with vehicle (n = 24). The study design is outlined in Table 9.

**Table 9: Study Design**

| Surgical Model | Treatment | N | Sex Species Age | Dosing Regimen |
|---|---|---|---|---|
| Sham | Vehicle | 24 | Sprague-Dawley Rats 20 weeks | 6 weeks daily SC treatment |
| OVX | Vehicle | 20 | | |
| OVX | abaloparatide 5 u2:/kg | 20 | | |
| OVX | abaloparatide 20 u2:/kg | 21 | | |

Bone densitometry (BMD) was measured *in vivo* by dual energy x-ray adsorptiometry (DXA) at baseline and end of study at six weeks. Animals were then euthanized and the femurs and L4 vertebrae were collected, wrapped with ethanol-soaked gauze and frozen at -20°C for high resolution CT (µCT) and biomechanical testing.

### Bone densitometry by dual energy x-ray

Rats were anesthetized with isoflurane and DXA (PIXImus, GE-Lunar Corporation, Fitchburg, WI) was used to measure in vivo bone mineral density (BMD) (grams per square centimeter) of the forth lumbar vertebrae (L4) and whole femur. BMD was measured at baseline and at the end of the 6-week dosing period.

### Microcomputed tomography (µCT) Measurements

Quantitative microcomputed tomography (mCT40 µCT scanner, Scanco Medical AG, Basserdorf, Switzerland) was used *ex vivo* to assess trabecular bone morphology in the forth-lumbar vertebrae and distal femoral metaphysis, and cortical bone geometry at the midfemoral diaphysis.

Scanning for the trabecular bone at the distal femoral metaphysis was initiated proximally at the level of the growth plate and extended distally 250 slices. Evaluations were performed on 150 slices beginning from ~0.2 mm distal to the growth plate. The entire L4 vertebrae was scanned, and the trabecular bone within the cranial and caudal growth plates and the cortex was evaluated. Morphometric parameters, including bone volume fraction (BV/TV, %), bone volume (BV, mm³), total volume (TV, mm³), trabecular number (Tb.N, 1/mm), trabecular thickness (Tb.Th, mm), trabecular spacing (Tb.Sp,mm), connectivity density (Conn.D, 1/mm³), structural model index (SMI) and bone density (BD, mg/mm²). At the femoral midshaft (cortical bone), 23 transverse CT slices were obtained and used to compute the total volume (TV, mm³), cortical bone volume (BV, mm³), marrow volume (MV, mm³), cortical thickness (Cort.Th, mm), and bone volume fraction (BV/TV, %).

### Biomechanical testing

Vertebrae bones (L4) were mechanically assayed by a compression test. Freshfrozen vertebrae were thawed to room temperature then the posterior pedicle arch, spinous process, and cranial and caudal ends were removed to obtain a vertebral body specimen with two parallel surfaces and a height approximately equal to 4 mm. Width in the medial-lateral and anterior-posterior directions at both the cranial and caudal ends was measured for the calculation of cross-sectional area. Vertebrae were placed between two platens and a load applied at a constant displacement rate of 6 mm/min until failure in an Instron Mechanical Testing Instrument (Instron 4465 retrofitted to 5500). The load and displacement curve was recorded by instrument software (Bluehill v2.5, Instron). The locations for maximum load at failure, stiffness and energy absorbed were selected manually from the load and displacement curve and calculated by instrument software (Bluehill v2.5, Instron). The intrinsic properties, ultimate strength, elastic modulus and toughness, were calculated from maximum load (N), stiffness (N/mm), energy absorbed (mJ), cross-sectional area and height (mm).

pQCT was performed on the excised right femurs using a Stratec XCT-RM and associated software (Stratec Medizintechnik GmbH, Pforzheim, Germany; software version 5.40). The scan was performed at 50% of the total femoral length from the distal end of the femur. The positions were verified using scout views and one 0.5-mm slice perpendicular to the long axis of the femoral shaft was acquired from each site. The scans were analyzed using a threshold for delineation of the external boundary. Axial area moment of inertia obtained from the pQCT scan was used in the calculation of intrinsic strength parameters at the femoral shaft.

For a three point bending test of the femoral shaft, each right femur was placed on the lower supports of a three point bending fixture with the anterior side facing downward in an Instron Mechanical Testing Instrument (Instron 4465 retrofitted to 5500). The span between the two lower supports was set at 14 mm. The upper loading device was aligned to the center of the femoral shaft. The load was applied at a constant displacement rate of 6 mm/min until the femur broke. The locations of maximum load, stiffness and energy absorbed were selected manually from the load and displacement curve and values calculated by instrument software (Bluehill v2.5, Instron). The intrinsic properties, ultimate strength, elastic modulus and toughness, were calculated from maximum load (N), stiffness (N/mm), energy absorbed (mJ), anterior-posterior diameter (mm) and moment of inertia (mm⁴).

For cantilever compression test of the femoral neck the proximal half of the femur was placed firmly in an anchoring platform where the greater trochanter was lodged in a notch cut in the platform. The test was conducted with an Instron Mechanical Testing Instrument (Instron 4465 retrofitted to 5500). The load was applied to the femoral head with a stainless steel probe, parallel to the femoral shaft at a constant displacement rate of 6 mm/min until failure. The locations of maximum load (N), stiffness (N/mm) and energy absorbed (mJ) were selected manually from the load and displacement curve and calculated by instrument software (Bluehill v2.5, Instron).

### Statistical analysis

Results are expressed as mean and standard deviation. Statistical analysis was performed using ANOVA followed by Tukey's multiple comparison test (Graphpad Instat, Cary, NC; release 9.1). All comparisons made in the text are statistically significant (p<0.05) unless otherwise stated.

### Results

### Bone Mineral Density

At the end of the bone depletion period, whole femur BMD was significantly decreased in OVX rats compared to Sham rats (11%, *p*<0.001 vs. Sham, data not shown). BMD values in OVX treated controls rats remained decreased compared to intact sham rats after 6 weeks of treatment (14% decrease, p<0.001 vs. Sham).

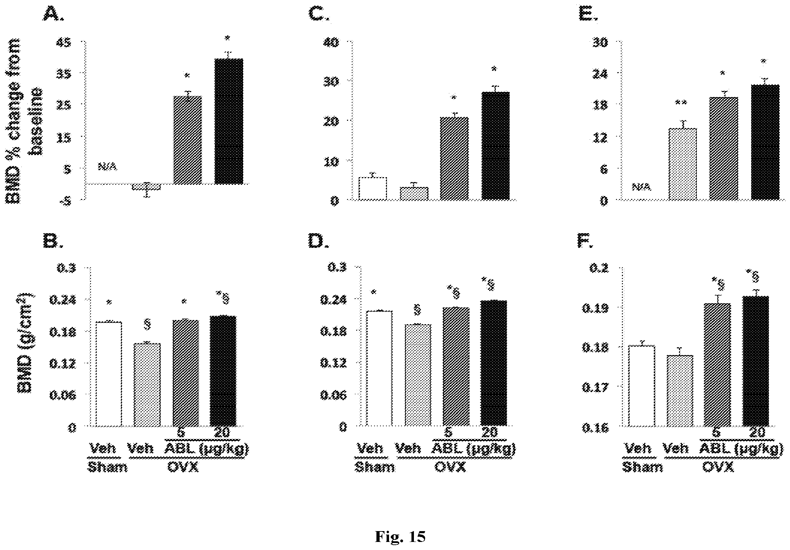

The bone mineral density (BMD) was measured by DXA at baseline (before dose initiation) and after 6 weeks of daily treatment with vehicle or abaloparatide. Compared to baseline, treatment of OVX rats with abaloparatide 5 µg/kg or abaloparatide 20 µg/kg resulted in significant increases in BMD at the spine (27% and 39% respectively, p<0.001 vs. baseline, Fig. 15A). Six weeks of treatment with abaloparatide led to marked dose-dependent increases in vertebral BMD versus OVX-Veh (28% and 33%, for abaloparatide 5 µg/kg and abaloparatide 20 µg/kg respectively, p<0.001 vs OVX-Veh, Fig. 15B). Abaloparatide treatment, not only restored OVX-induced bone loss, but treatment with abaloparatide 20 µg/kg increased BMD to levels above those of Sham control values (*p*<0.001 vs Sham).

Whole femur BMD was increased significantly and dose dependently with abaloparatide 5 µg/kg and abaloparatide 20 µg/kg over baseline by 21% and 27%, respectively (*p*<0.001 vs baseline, Fig. 15C). Similar increases in BMD from baseline were observed at the femur diaphysis (Fig. 15E). Abaloparatide treatment resulted in significant dose dependent gains in BMD for the total femur and at the femoral midshaft compared to OVX-Veh control rats as well as Sham control rats (*p*<0.001 vs OVX-Veh, *p*<0.001 vs Sham, Figs. 14D and 14F). Collectivity, these data demonstrated marked gains in bone mass in response to abaloparatide treatment.

### Bone microarchitecture

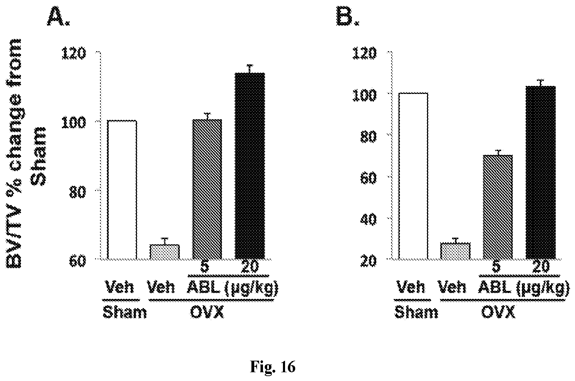

Consistent with the BMD measurements, OVX was associated with significant bone deterioration, particularly in the trabecular compartment (Fig. 16, Tables 7 and 8). Compared to Sham control rats, OVX-Veh rats had 36% lower BV/TV in the vertebral trabecular bone (Fig. 16A, Table 8, *p*<0.001 vs Sham). Additionally, Tb.N, Tb.Th and BD were lower together with higher Tb.Sp in the vertebral bone of OVX-Veh rats compared to Sham control rats (Table 10).

**Table 10: Effect of OVX and abaloparatide treatment on L4 lumbar spine, assessed by µCT**

| | SHAM Vehicle | OVX Vehicle | OVX Abaloparatide | |
|---|---|---|---|---|
| | | | 5 µg/kg | 20 µg/kg |
| L4 Lumbar Spine | | | | |
| BV/TV (%) | 51.5 ± 4.3^{∗∗∗} | 33.0 ± 0.5^{§§§} | 51.7 ± 4.7^{∗∗∗} | 58.6 ± 5.3^{∗∗∗§§§} |
| TV (mm³) | 30.4 ± 3.1 | 33.0 ± 4.1 | 32.3 ± 5.3 | 30.7 ± 4.6 |
| BV (mm3) | 15.7 ± 1.9^{∗∗∗} | 10.9 ± 2.1^{§§§} | 16.6 ± 2.7^{∗∗∗§§} | 18.0 ± 2.8^{∗∗∗} |
| Tb.Th (mm) | 0.110 ± 0.01^{∗∗∗} | 0.095 ± 0.01^{§§§} | 0.136 ± 0.01^{∗∗∗§§§} | 0.152 ± 0.01^{∗∗∗§§§} |
| Tb.N (1/mm) | 4.87 ± 0.28^{∗∗∗} | 3.62 ± 0.48^{§§§} | 3.91 ± 0.30^{∗§§§} | 4.05 ± 0.27^{∗∗∗§§§} |
| Tb.Sp (mm) | 0.181 ± 0.01^{∗∗∗} | 0.268 ± 0.05^{§§§} | 0.219 ± 0.03^{∗∗∗§§§} | 0.201 ± 0.02^{∗∗∗§§§} |
| Conn.D (1/mm³) | 75.0 ± 12.9 | 68.3 ± 11.3 | 48.0 ± 5.4^{∗∗∗§§§} | 42.1 ± 7.2^{∗∗∗§§§} |
| SMI | -1.82 ± 0.74^{∗∗∗} | 0.29 ± 0.43^{§§§} | -1.33 ± 0.56^{∗} | -2.23 ± 0.92^{∗∗∗§§§} |
| BD (mg/mm²) | 560 ± 35^{∗∗∗} | 394 ± 51^{§§§} | 570 ± 46^{∗∗∗} | 631 ± 50^{∗∗∗§§§} |

| | | | | |
|---|---|---|---|---|
| Data are mean ± standard deviation. n = 20-24 per treatment group. BV/TV, Bone volume fraction; TV, Total volume; BV, Bone volume; MV, marrow volume; Ct.Th, cortical thickness, Tb.Th, trabecular thickness; Tb.N, trabecular number; Tb.Sp, trabecular separation; Conn.D, Connectivity density; SMI, Structure model index; BD, bone density. *p* vs. vehicle treated OVX rats: ^{∗}*p*≤0.05; ^{∗∗}*p*<0.01; ^{∗∗∗}*p*<0.001. *p* vs. vehicle treated Sham rats: ^{§}*p*≤0.05; ^{§ §}*p*<0.01; ^{§ § §}*p*<0.001. Bolded p abaloparatide 20 µg/kg vs. abaloparatide 5 µg/kg treated OVX rats: p<0.05. | | | | |

At the trabecular compartment of the distal femur, BV/TV was 71% lower in OVXVeh rats relative to Sham rats (Fig. 16B, Table 9, *p*<0.001 vs Sham). Compared to Sham control rats, Tb.N, Tb.Th, and Conn.D were lower in OVX-Veh rats (Table 11). Cortical bone was also decreased by OVX, with BV/TV and Ct.Th significantly lower in the femur diaphysis of OVX-Veh rats than Sham control rats (Table 13, *p*<0.01 vs Sham).

**Table 11: Effect of OVX and abaloparatide treatment on the distal femoral trabecular bone and femoral diaphysis. assessed by µCT**

| | SHAM Vehicle | OVX Vehicle | OVX Abaloparatide | |
|---|---|---|---|---|
| | | | 5 µg/kg | 20 µg/kg |

| Femoral trabecular bone | | | | |
|---|---|---|---|---|
| BV/TV (%) | 53.0 ± 9.2^{∗∗∗} | 15.2 ± 4.5^{§§§} | 37.2 ± 6.5^{∗∗∗sss} | 56.2 ± 7.9^{∗∗∗} |
| TV (mm³) | 30.2 ± 2.8 | 28.9 ± 2.8 | 28.9 ± 3.5 | 29.2 ± 3.6 |
| BV (mm³) | 16.11 3.8^{∗∗∗} | 4.43 ± 1.5^{§§§} | 10.76 ± 2.4^{∗∗∗§§§} | 16.58 3.8^{∗∗∗} |
| Tb.Th (mm) | 0.119 ± 0.02^{∗∗∗} | 0.087 ± 0.01^{§§§} | 0.128 ± 0.01^{∗∗∗§§§} | 0.186 ± 0.03^{∗∗∗§§§} |
| Tb.N (1/mm) | 5.74 ± 0.62^{∗∗∗} | 1.66 ± 0.59^{§§§} | 2.43 ± 0.66^{∗∗∗§§§} | 3.01 ± 0.52^{∗∗∗§§§} |
| Tb.Sp (mm) | 0.147 ± 0.03^{∗∗∗} | 0.715 ± 0.28^{§§§} | 0.494 ± 0.17^{∗∗§§§} | 0.399 ± 0.11^{∗∗∗§§§} |
| Conn.D (1/mm³) | 115.9 ± 19.5^{∗∗∗} | 42.7 ± 12.8^{§§§} | 53.1 ± 10.6^{∗∗∗§§§} | 34.7 ± 9.0^{∗§§§} |
| SMI | -1.67 ± 2.31^{∗∗∗} | 1.58 ± 0.17^{§§§} | -0.39 ± 0.46^{∗∗∗§} | -3.26 ± 1.73^{∗∗∗§§} |
| BD (mg/mm²) | 575 ± 79^{∗∗∗} | 199 ± 56^{§§§} | 421 ± 65^{∗∗∗§§§} | 596 ± 84^{∗∗∗} |

| Femoral cortical bone | | | | |
|---|---|---|---|---|
| BV/TV (%) | 67.3 3^{∗∗} | 66.3 ± 2^{§§} | 66.8 ± 4^{§§§} | 70.0 ± 3^{∗§§§} |
| TV (mm³) | 3.97 ± 0.28 | 4.13 ± 0.30 | 4.48 ± 0.43^{∗§§§} | 4.35 ± 0.49^{§§} |
| BV (mm³) | 2.67 ± 0.14 | 2.74 ± 0.18 | 2.98 ± 0.18^{∗∗§§§} | 3.04 ± 0.28^{∗∗∗§§§} |
| MV (mm³) | 1.30 ± 0.19 | 1.39 ± 0.17 | 1.50 ± 0.29 | 1.32 ± 0.26 |
| Ct.Th (mm) | 0.616 ± 0.08^{∗∗} | 0.674 ± 0.04^{∗∗} | 0.703 ± 0.04^{§§§} | 0.723 ± 0.05^{∗§§§} |

| | | | | |
|---|---|---|---|---|
| Data are mean ± standard deviation. n = 20-24 per treatment group. BV/TV, Bone volume fraction; TV, Total volume; BV, Bone volume; MV, marrow volume; Ct.Th, cortical thickness, Tb.Th, trabecular thickness; Tb.N, trabecular number; Tb.Sp, trabecular separation; Conn.D, Connectivity density; SMI, Structure model index; BD, bone density. *p* vs. vehicle treated OVX rats: ^{∗}*p*≤0.05; ^{∗∗}*p*<0.01; ^{∗∗∗}*p*<0.001. *p* vs. vehicle treated Sham rats: ^{§}*p*≤0.05; ^{§ §}*p*<0.01; ^{§ § §}*p*<0.001. Bolded p abaloparatide 20 µg/kg vs. abaloparatide 5 µg/kg treated OVX rats: p<0.05. | | | | |

Six weeks treatment with abaloparatide improved bone microarchitectural properties in OVX rats and fully inhibited OVX-induced bone loss, improving cortical and trabecular bone parameters to levels at or above the OVX-Veh and Sham-Veh-treated rats. Specifically, abaloparatide 20 µg/kg-treated animals had significantly higher BV/TV in the vertebral trabecular bone compartment compared to OVX-Veh animals (77%, *p*<0.001 vs OVX-Veh, Fig. 16A, Table 10) and Sham-Veh animals (14%,p<0.001 vs OVX-Veh, Fig. 16A, Table 10); and abaloparatide 5 µg/kg treatment increased BV/TV by 56% over OVXVeh treatment (*p*<0.001 vs OVX-Veh). At the trabecular bone of the distal femur, abaloparatide 5 µg/kg and abaloparatide 20 µg/kg treatment increased BV/TV by approximately 2.5- and 3.7-fold, respectively, over OVX-Veh (*p*<0.001 vs OVX-Veh, Fig. 16B, Table 11). Tb.Th, Tb.N along with lower Tb.Sp, better connectivity density, and more plate-like architecture (SMI) were significantly improved compared to Vehicle-treated animals at the femur (Table 11). In addition, six week of treatment with abaloparatide 20 µg/kg improved femur midshaft properties in OVX animals, significantly increasing bone volume fraction (BV/TV) by 6% and 4% compared to OVX-Veh treatment (p<0.05 vs OVXVeh, Table 11) and Sham-Veh control, respectively, (*p*<0.001 vs Sham, Fig. 16B, Table 11).

Treatment of abaloparatide 20 µg/kg also led to increase in cortical thickness compared to OVXVeh treatment (*p*<0.05, Table 11).

### Vertebral and femoral bone strength

L4 maximum load and ultimate strength were ~28% lower in OVX-Veh rats compared to Sham control rats (p<0.01, Table 12). Compression testing of L4 showed that rats treated with abaloparatide 5 µg/kg and abaloparatide 20 µg/kg had significantly higher mechanical testing values compared to OVX-Veh treated rats control with maximum load (170% and 180%, *p*<0.05 and 0.01 vs. OVX-Veh, respectively, Table 12), energy absorbed (280% and 290%, *p*<0.001), ultimate strength (170% and 180%, p<0.001) and toughness (270%, both groups, *p*<0.001). Further, significant increases in maximum load (126%, *p*<0.05) and toughness (170%, *p*<0.01) of the L4 vertebra were seen in OVX rats treated with abaloparatide 20 µg/kg versus Sham control rats.

**Table 12: Effect of OVX and abaloparatide treatment on L4 lumbar spine, assessed by biomechanical testing**

| | SHAM Vehicle | OVX Vehicle | OVX Abaloparatide | |
|---|---|---|---|---|
| | | | 5 µg/kg | 20 µg/kg |

| Vertebral compression | | | | |
|---|---|---|---|---|
| Maximum Load (N) | 265 ± 81^{∗∗} | 190 ± 71^{§§} | 323 ± 68^{∗∗∗§} | 336 ± 76^{∗∗∗§§} |
| Stiffness (N/mm) | 2032 ± 913 | 1795 ± 894 | 1872 ± 1037 | 1845 ± 954 |
| Energy (mJ) | 35 ± 9^{∗∗} | 22 ± 12^{§§} | 62 ± 38^{∗∗∗§§} | 64 ± 29^{∗∗∗§§§} |
| Ult. Strength (N/mm²) | 34 ± 9^{∗∗∗} | 24 ± 8^{§§§} | 40 ± 9^{∗∗∗§} | 41 ± 9^{∗∗∗§§} |
| Elastic Modulus (MPa) | 1052 ± 445 | 930 ± 437 | 963 ± 565 | 941 ± 509 |
| Toughness (MJ/m³) | 1.09 ± 0.46^{∗∗∗} | 0.66 ± 0.32^{§§§} | 1.86 ± 1.04^{∗∗∗§§} | 1.85 ± 0.62^{∗∗∗§§§} |

| | | | | |
|---|---|---|---|---|
| Data are mean ± standard deviation. n = 20-24 per treatment group. *p* vs. vehicle treated OVX rats: ^{∗}*p*≤0.05; ^{∗∗}*p*<0.01; ^{∗∗∗}*p*<0.001. *p* vs. vehicle treated Sham rats: ^{§}*p*≤0.05; ^{§ §}*p*<0.01; ^{§ § §}*p*<0.001. | | | | |

Strength parameters of femurs from OVX-Veh rats tended to be higher than Sham rats, with maximum load, energy and toughness parameters were 8%, 25% and 18%, respectively, higher in OVX-Veh rats (*p*<0.05 vs Sham, Table 13).

**Table 13: Effect of OVX and abaloparatide treatment on the femur, assessed by biomechanical testing**

| | SHAM Vehicle | OVX Vehicle | OVX Abaloparatide | |
|---|---|---|---|---|
| | | | 5 µg/kg | 20 µg/kg |

| Three Point Bending Test of the Femur | | | | |
|---|---|---|---|---|
| Maximum Load (N) | 188 ± 14^{∗∗∗} | 204 ± 21^{§§§} | 223 ± 16^{∗∗§§§} | 224 ± 25^{∗∗§§§} |
| Stiffness (N/mm) | 771 ± 105 | 779 ± 133 | 874 ± 120^{∗§§} | 872 ± 127^{∗§§} |
| Energy (mJ) | 56 ± 16^{∗} | 71 ± 19^{§} | 78 ± n^{§§§} | 76 ± 20^{§§§} |
| Ult. Strength (N/mm²) | 173 ± 16 | 176 ± 15 | 185 ± 18^{§} | 184 ± 19^{§} |
| Elastic Modulus (MPa) | 7479 ± 1113 | 7100 ± 1173 | 7381 ± 1502 | 7449 ± 1480 |
| Toughness (MJ/m³) | 4.9 ± 1.5^{∗} | 5.8 ± 1.4^{§} | 6.3 ± 1.3^{§§§§} | 6.0 ± 1.4^{§} |
| AP Diameter (mm) | 3.1 ± 0.1 | 3.1 ± 0.1 | 3.2 ± 0.1^{∗∗§§} | 3.2 ± 0.2 |
| AAMI (mm4) | 5.9 ± 0.7 | 6.3 ± 0.8 | 6.9 ± 1.0^{∗§§§} | 6.9 ± 1.3^{§§} |

| Cantilever Compression, Femoral Neck | | | | |
|---|---|---|---|---|
| Maximum Load (N) | 100 ± 13 | 93 ± 15 | 123 ± 25^{∗∗∗§§§} | 116 ± 20^{∗∗§§} |
| Stiffness (N/mm) | 216 ± 55 | 189 ± 55 | 226 ± 65 | 198 ± 56 |
| Energy (mJ) | 31 ± 10 | 34 ± 11 | 46 ± 25^{§} | 46 ± 14^{∗∗§§§} |

| | | | | |
|---|---|---|---|---|
| Data are mean ± standard deviation. n = 20-24 per treatment group. Ult. Strength = ultimate strength; AP Diameter = anterior-posterior diameter; AAMI = axial area of the moment inertia *p* vs. vehicle treated OVX rats: ^{∗}*p*≤0.05; ^{∗∗}*p*<0.01; ^{∗∗∗}*p*<0.001. *p* vs. vehicle treated Sham rats: ^{§}*p*≤0.05; ^{§ §}*p*<0.01; ^{§ § §}*p*<0.001. | | | | |

Strength parameters of femurs in OVX-Veh rats that are higher than Sham control measured in the first 1-12 weeks from baseline have been reported previously in OVX rats (6). abaloparatide 5 µg/kg and abaloparatide 20 µg/kg treatment further improved mechanical properties of the femur bone compared to OVX control rats, with maximum load (110%, *p*<0.05 vs OVX-Veh, Table 13), ultimate strength (158%, *p*<0.001 vs OVX-Veh) and the axial area of moment of inertia (110%, *p*<0.001 vs OVX-Veh) higher than OVX-Veh control. Additionally, treatment with abaloparatide 5 µg/kg and abaloparatide 20 µg/kg improved mechanical properties of the femur compared to Sham control rats, with maximum load (19%, *p*<0.001 vs OVXVeh, Table 13), stiffness (13%, *p*<0.01 vs OVX-Veh), energy (34% and 37%, respectively, *p*<0.001 vs OVX-Veh), ultimate strength (7%, *p*<0.05 vs OVXVeh), toughness (22% and 29%, respectively, p<0.05 vs OVX-Veh), and the axial area of moment of inertia (15%, p<0.01 vs OVX-Veh) higher than OVX-Veh control Cantilever compression of the femoral neck showed that the maximum load tolerated was 108% lower in OVX-Veh treated rats than Sham rats (*p*<0.01 vs Sham, Table 13). OVX rats treated with abaloparatide 5 µg/kg and abaloparatide 20 µg/kg demonstrated increased strength of the femoral neck, with maximum load (23% and 16%, respectively, *p*<0.01 vs OVX-Veh, Table 13), and energy (48%, *p*<0.05 vs OVX-Veh) higher than OVX-Veh control. Together, consistent with increases in BMD and bone microarchitecture, the data demonstrated that abaloparatide treatment improved bone strength parameters in OVX rats.

### Discussion

The bone anabolic effect of six weeks of daily administration of abaloparatide, an example of synthetic PTHrP analog, in adult ovariectomized osteopenic rats were assessed. The results showed that abaloparatide treatment reversed bone loss and the deterioration of bone mechanical properties associated with OVX-induced osteopenia with promoted gains in bone mass and restoration of bone microarchitecture. Treatment with abaloparatide reversed bone mass and restored bone quality as demonstrated by increases in BMD, trabecular and cortical microarchitecture, and femoral neck and diaphysis strength values in the OVX rats treated with abaloparatide, compared with OVX-Veh rats after 6 weeks of treatment. Furthermore, treatment with abaloparatide resulted in values that were at or above the Sham-Vehicle control group. These observations of marked bone anabolic activity following treatment with abaloparatide in a rat OVX-induced osteoporosis model are consistent with the BMD gains seen in a effects of abaloparatide treatment in postmenopausal woman with osteoporosis (e.g., Example 1).

The results of this study demonstrate that six weeks of treatment with abaloparatide induced a marked and dose-dependent increase in BMD of the trabecular bone compartment at the lumbar spine (28 % and 33 %, for abaloparatide 5 µg/kg and 20 µg/kg, respectively) and femoral bone (17 % and 23 %, abaloparatide 5 µg/kg and 20 µg/kg, respectively) compared to OVX-Vehicle control rats. Assessment of trabecular bone microarchitecture provided further insight into the nature of abaloparatide-induced BMD gains. A dose dependent increases for abaloparatide 5 µg/kg and abaloparatide 20 µg/kg was observed in bone volume fraction (BV/TV) at the vertebral trabecular bone (57% and 78%, respectively) and the trabecular bone of the distal femur (145% and 270%, respectively).

These increases were related to increases in trabecular thickness, trabecular number, accompanied by concomitant decrease in trabecular separation compared to OVX-vehicle treated rats. These gain in bone mass and increases in bone microarchitecture parameters in the trabecular bone compartment were associated with increased biomechanical parameters. After 6 weeks of treatment bone mass, microarchitecture, and biomechanics were normalized for most parameters compared to sham controls and many parameters were significantly increased relative to Sham. These findings are consistent with recently reported clinical study results where abaloparatide treatment increase BMD in lumbar spine and hip as early as 12 weeks of treatment in woman with osteoporosis (see, e.g., Example 1). As shown in Example 1, BMD gains were greater than observed with teriparatide (rhPTH(1-34)) at both the 12 and 24-week time points. The increase in lumbar spine BMD with abaloparatide was markedly greater than that observed with teriparatide 20 µg and were comparable to previously reported PTH induced bone gains in clinical studies (37).

The effects of abaloparatide treatment were seen in all regions of the femur suggesting that the effect on BMD potentially includes positive effects on both the trabecular and cortical bone compartments. Indeed, cortical bone exhibited approximately 8% increase in bone mass after six weeks of abaloparatide 5 µg/kg and 20 µg/kg treatment in OVX rats compared to OVX-vehicle treated rats. The physiological mechanisms underlying the BMD effects in the cortical bone observed with abaloparatide treatment are not entirely clear. Example 1 showed significant increases in total hip BMD with abaloparatide treatment compared to teriparatide treatment. The higher ratio of formation versus resorption in abaloparatide-treated women may be a contributing factor to the differential effects of these two agents on BMD. Prior studies reported that treatment with PTH in OVX monkeys increased cortical porosity in the humerus (38).

Moreover, clinical studies suggested that PTH early effects at cortical sites are to increase intracortical bone remodeling, leading to increased cortical porosity (29,31,32,39,37). It was further suggested that the increase in the rate of bone resorption following abaloparatide treatment is more limited and delayed compared to PTH, it is possible that gains in cortical BMD are also the result of an absolute lower rate of intracortical resorption hence less cortical porosity. Additional experimental studies that assessed the effect of abaloparatide on cortical porosity would provide further insight into the effect on cortical bone. The current study, also demonstrated abaloparatide-induced increases in cortical BMD, along with increases in trabecular bone microarchitecture parameters, where associated with increases in bone strength. Altogether, these increases in bone parameters suggested a positive effect on bone quality.

The molecular mechanisms by which abaloparatide exerts its anabolic action are not fully understood but may have some similarities to the parent protein, PTHrP. PTH and PTHrP share some sequence homology and may have arisen by duplication of a common ancestral gene, but each plays a distinct role in bone physiology. PTH, secreted by the parathyroid glands, acts in a classical endocrine manner to promote osteoclastic bone resorption and calcium mobilization. In contrast, PTHrP functions as a paracrine regulator of bone formation. Despite these differences, PTH and PTHrP both increase intracellular cAMP concentrations by activating the same PTH/PTHrP receptor type 1 (PTHR), a G proteincoupled receptor (GPCR). However, continuous administration of PTH leads to bone resorption over formation, whereas continuous PTHrP administration preferentially stimulates formation (40,41). Recent studies have provided a basis for the divergent actions of PTH and PTHrP in bone. Specifically, PTHrP activity at the PTHR is restricted to the cell surface and yields a brief intracellular cAMP burst. Whereas, the conformation associated with PTH stabilizes its binding to the receptor and its coupled G-protein and moves to internalized compartments of the cell, and leads to persistent cAMP generation (12,14,42,43). The significance of ligands that form more stable complexes and more cAMP responses a more catabolic response resulting in elevated blood calcium levels (13). In contrast, ligands such as PTHrP transiently producing cAMP and mobilizing calcium, yet results in greater anabolic action than PTH.

Consistent with these reports, a recent study evaluated the binding of abaloparatide to two distinct PTHR1 conformations. The findings suggested that the enhanced bone anabolic activity seen with abaloparatide treatment may arise from a more selective binding to the RO PTHR1 than the RG conformation, compared to PTH long-acting PTH (LA-PTH) or PTHrP (44). Further studies will be required to elucidate the molecular mechanisms of abaloparatide that are resulting in increased anabolic activity.

In summary, 6 weeks of abaloparatide treatment in OVX osteopenic rats, increased bone mass and microarchitecture parameters that resulted in increased bone strength. This capacity to increase BMD along with improvements in bone quality in this preclinical model highlights the bone anabolic activity of abaloparatide, and support the continued investigation of abaloparatide as potential therapy for the treatment of postmenopausal osteoporosis.

### REFERENCES

1. 2004 Bone health and osteoporosis: A report of the Surgeon General. In: United States Department of Health and Human Services PHS ed.: Office of the Surgeon General.
2. Papapoulos SE 2011 Use of bisphosphonates in the management of postmenopausal osteoporosis. Ann NY Acad Sci 1218:15-32.
3. Cosman F 2008 Parathyroid hormone treatment for osteoporosis. Curr Opin Endocrinol Diabetes Obes 15:495-501.
4. MacLean C, Newberry S, Maglione M, McMahon M, Ranganath V, Suttorp M, Mojica W, Timmer M, Alexander A, McNamara M, Desai SB, Zhou A, Chen S, Carter J, Tringale C, Valentine D, Johnsen B, Grossman J 2008 Systematic review: comparative effectiveness of treatments to prevent fractures in men and women with low bone density or osteoporosis. Ann Intern Med 148:197-213.
5. Recker RR, Marin F, Ish-Shalom S, Moricke R, Hawkins F, Kapetanos G, de la Pena MP, Kekow J, Farrerons J, Sanz B, Oertel H, Stepan J 2009 Comparative effects of teriparatide and strontium ranelate on bone biopsies and biochemical markers of bone turnover in postmenopausal women with osteoporosis. J Bone Miner Res 24:1358-1368.
6. Dempster DW, Zhou H, Recker RR, Brown JP, Bolognese MA, Recknor CP, Kendler DL, Lewiecki EM, Hanley DA, Rao DS, Miller PD, Woodson GC, 3rd, Lindsay R, Binkley N, Wan X, Ruff VA, Janos B, Taylor KA 2012 Skeletal histomorphometry in subjects on teriparatide or zoledronic acid therapy (SHOTZ) study: a randomized controlled trial. J Clin Endocrinol Metab 97:2799-2808.
7. Ma YL, Marin F, Stepan J, Ish-Shalom S, Moricke R, Hawkins F, Kapetanos G, de la Pena MP, Kekow J, Martinez G, Malouf J, Zeng QQ, Wan X, Recker RR 2011 Comparative effects of teriparatide and strontium ranelate in the periosteum of iliac crest biopsies in postmenopausal women with osteoporosis. Bone 48:972-978.
8. Leder BZ, Tsai JN, Uihlein AV, Burnett-Bowie SA, Zhu Y, Foley K, Lee H, Neer RM 2014 Two Years of Denosumab and Teriparatide Administration in Postmenopausal Women with Osteoporosis (The DATA Extension Study): a Randomized Controlled Trial. J Clin Endocrinol Metab:jc20134440.
9. Neer RM, Arnaud CD, Zanchetta JR, Prince R, Gaich GA, Reginster JY, Hodsman AB, Eriksen EF, Ish-Shalom S, Genant HK, Wang O, Mitlak BH 2001 Effect of parathyroid hormone (1-34) on fractures and bone mineral density in postmenopausal women with osteoporosis. N Engl J Med 344:1434-1441.
10. Dhainaut A, Hoff M, Syversen U, and Haugeberg G. Cortical hand bone porosity and its association with distal radius fracture in middle aged and elderly women. PLOS 2013; 7:1-6.
11. Kronenberg HM 2006 PTHrP and skeletal development. Ann N Y Acad Sci 1068:1-13.
12. Pioszak AA, Parker NR, Gardella TJ, Xu HE 2009 Structural basis for parathyroid hormone-related protein binding to the parathyroid hormone receptor and design of conformation-selective peptides. J Biol Chem 284:28382-28391.
13. Okazaki M, Ferrandon S, Vilardaga JP, Bouxsein ML, Potts JT, Jr., Gardella TJ 2008 Prolonged signaling at the parathyroid hormone receptor by peptide ligands targeted to a specific receptor conformation. Proc Natl Acad Sci U S A 105:16525-16530.
14. Dean T, Vilardaga JP, Potts JT, Jr., Gardella TJ 2008 Altered selectivity of parathyroid hormone (PTH) and PTH-related protein (PTHrP) for distinct conformations of the PTH/PTHrP receptor. Mol Endocrinol 22:156-166.
15. Horwitz MJ, Augustine M, Khan L, Martin E, Oakley CC, Carneiro RM, Tedesco MB, Laslavic A, Sereika SM, Bisello A, Garcia-Ocana A, Gundberg CM, Cauley JA, Stewart AF 2013 A comparison of parathyroid hormone-related protein (1-36) and parathyroid hormone (1-34) on markers of bone turnover and bone density in postmenopausal women: the PrOP study. J Bone Miner Res 28:2266-2276.
16. Horwitz MJ, Tedesco MB, Garcia-Ocana A, Sereika SM, Prebehala L, Bisello A, Hollis BW, Gundberg CM, Stewart AF 2010 Parathyroid hormone-related protein for the treatment of postmenopausal osteoporosis: defining the maximal tolerable dose. J Clin Endocrinol Metab 95:1279-1287.
17. Horwitz MJ, Tedesco MB, Sereika SM, Garcia-Ocana A, Bisello A, Hollis BW, Gundberg C, Stewart AF 2006 Safety and tolerability of subcutaneous PTHrP(1-36) in healthy human volunteers: a dose escalation study. Osteoporos Int 17:225-230.
18. Obaidi M, Chavira RE, Reinbolt L, Offman E, McKay E, O'Dea LL 2010 Pharmacokinetics and Pharmacokinetics and pharmacodynamic of subcutaneously (SC) administered doses of BA058, a bone mass density restoring agent in healthy postmenopausal women. In: AAPS(abstract); W5385.
19. Doyle N, Varela A, Smith SY, Guldberg R, Hattersley G 2013 Long Term effect of BA058, a Novel Human PTHrP Analog, Restores Bone Mass in the Aged Osteopenic Ovariectomized Cynomolgus Monkey. J Bone Miner Res 28(Suppl 1)(abstract)
20. Hattersley G, Lesage E, Varela A, Mith SY 2013 BA058, a Novel Human PTHrP Analog, Restores Bone Density and Increases Bone Strength At the Spine and Femur in Osteopenic Rats. Endocr Rev 34(abstract).
21. Oei et al., High bone mineral density and fracture risk in type 2 diabetes as skeletal complications of inadequate glucose control.
22. Bonnick SL, Johnston CC, Jr., Kleerekoper M, Lindsay R, Miller P, Sherwood L, Siris E 2001 Importance of precision in bone density measurements. J Clin Densitom 4:105-110.
23. Bonnick S, Saag KG, Kiel DP, McClung M, Hochberg M, Burnett SM, Sebba A, Kagan R, Chen E, Thompson DE, de Papp AE 2006 Comparison of weekly treatment of postmenopausal osteoporosis with alendronate versus risedronate over two years. J Clin Endocrinol Metab 91:2631-2637.
24. Gallagher JC, Rosen CJ, Chen P, Misurski DA, Marcus R 2006 Response rate of bone mineral density to teriparatide in postmenopausal women with osteoporosis. Bone 39:1268-1275.
25. Hochberg MC, Ross PD, Black D, Cummings SR, Genant HK, Nevitt MC, Barrett-Connor E, Musliner T, Thompson D 1999 Larger increases in bone mineral density during alendronate therapy are associated with a lower risk of new vertebral fractures in women with postmenopausal osteoporosis. Fracture Intervention Trial Research Group. Arthritis Rheum 42:1246-1254.
26. Miller PD, McClung MR, Macovei L, Stakkestad JA, Luckey M, Bonvoisin B, Reginster JY, Recker RR, Hughes C, Lewiecki EM, Felsenberg D, Delmas PD, Kendler DL, Bolognese MA, Mairon N, Cooper C 2005 Monthly oral ibandronate therapy in postmenopausal osteoporosis: 1-year results from the MOBILE study. J Bone Miner Res 20:1315-1322.
27. Sebba AI 2008 Significance of a decline in bone mineral density while receiving oral bisphosphonate treatment. Clin Ther 30:443-452.
28. Sebba AI, Bonnick SL, Kagan R, Thompson DE, Skalky CS, Chen E, de Papp AE 2004 Response to therapy with once-weekly alendronate 70 mg compared to once-weekly risedronate 35 mg in the treatment of postmenopausal osteoporosis. Curr Med Res Opin 20:2031-2041.
29. Hansen S, Hauge EM, Beck Jensen JE, Brixen K 2013 Differing effects of PTH 1-34, PTH 1-84, and zoledronic acid on bone microarchitecture and estimated strength in postmenopausal women with osteoporosis: an 18-month open-labeled observational study using HR-pQCT. J Bone Miner Res 28:736-745.
30. Tsai J, Uihlein A, Zhu Y, Foley K, Lee H, Burnett-Bowie SA, Neer R, Bouxsein M, Leder B 2013 Comparative effects of teriparatide, denosumab, and combination therapy on peripheral compartmental bone density and microarchitecture: the DATA-HRpQCT Study. In: Meeting of the American Society of Bone and Mineral Research. Baltimore, MD.
31. Nishiyama KK, Cohen A, Young P, Wang J, Lappe JM, Guo XE, Dempster DW, Recker RR, Shane E 2014 Teriparatide increases strength of the peripheral skeleton in premenopausal women with idiopathic osteoporosis: a pilot HR-pQCT study. J Clin Endocrinol Metab 99:2418-2425.
32. Ma YL, Zeng QQ, Chiang AY, Burr D, Li J, Dobnig H, Fahrleitner-Pammer A, Michalska D, Marin F, Pavo I, Stepan JJ 2014 Effects of teriparatide on cortical histomorphometric variables in postmenopausal women with or without prior alendronate treatment. Bone 59:139-147.
33. Keaveny TM, McClung MR, Wan X, Kopperdahl DL, Mitlak BH, Krohn K 2012 Femoral strength in osteoporotic women treated with teriparatide or alendronate. Bone 50:165-170.
34. Han SL, Wan SL 2012 Effect of teriparatide on bone mineral density and fracture in postmenopausal osteoporosis: meta-analysis of randomized controlled trials. Int J Clin Pract 66:199-209.
35. Silva et al., Trabecular bone score: a noninvasive analytical method based upon the DXA image, J. Bone Miner. Res. 29(3): 518-530 (2014).
36. Amugongo SK, Yao W, Jia J, Dai W, Lay YA, Jiang L, Harvey D, Zimmermann EA, Schaible E, Dave N, Ritchie RO, Kimmel DB, Lane NE. Effect of sequential treatments with alendronate, parathyroid hormone (1-34) and raloxifene on cortical bone mass and strength in ovariectomized rats. Bone 2014;67: 257-68.
37. Tsai JN, Uihlein AV, Lee H, Kumbhani R, Siwila---Sackman E, McKay EA, Burnett-Bowie SA, Neer RM, Leder BZ. Teriparatide and denosumab, alone or combined, in women with postmenopausal osteoporosis: the DATA study randomized trial. Lancet 2013;382: 50-6.
38. Burr DB, Hirano T, Turner CH, Hotchkiss C, Brommage R, Hock JM. Intermittently administered human parathyroid hormone(1---34) treatment increases intracortical bone turnover and porosity without reducing bone strength in the humerus of ovariectomized cynomolgus monkeys. J Bone Miner Res 2001;16: 157---65.
39. Tsai JN, Uihlein AV, Burnett-Bowie SA, Neer RM, Zhu Y, Derrico N, Lee H, Bouxsein ML, Leder BZ. Comparative effects of teriparatide, denosumab, and combination therapy on peripheral compartmental bone density, microarchitecture, and estimated strength: the DATA-HRpQCT Study. J Bone Miner Res 2015;30: 39-45.
40. Horwitz MJ, Tedesco MB, Sereika SM, Hollis BW, Garcia-Ocana A, Stewart AF. Direct comparison of sustained infusion of human parathyroid hormone-related protein-(1-36) [hPTHrP-(1-36)] versus hPTH-(1-34) on serum calcium, plasma 1,25-dihydroxyvitamin D concentrations, and fractional calcium excretion in healthy human volunteers. J Clin Endocrinol Metab 2003;88: 1603---9.
41. Horwitz MJ, Tedesco MB, Sereika SM, Syed MA, Garcia-Ocana A, Bisello A, Hollis BW, Rosen CJ, Wysolmerski JJ, Dann P, Gundberg C, Stewart AF. Continuous PTH and PTHrP infusion causes suppression of bone formation and discordant effects on 1,25(OH)2 vitamin D. J Bone Miner Res 2005;20: 1792-803.
42. Ferrandon S, Feinstein TN, Castro M, Wang B, Bouley R, Potts JT, Gardella TJ, Vilardaga JP. Sustained cyclic AMP production by parathyroid hormone receptor endocytosis. Nat Chem Biol 2009;5: 734-42.
43. Dean T, Linglart A, Mahon MJ, Bastepe M, Juppner H, Potts JT, Jr., Gardella TJ. Mechanisms of ligand binding to the parathyroid hormone (PTH)/PTH-related protein receptor: selectivity of a modified PTH(1-15) radioligand for GalphaS-coupled receptor conformations. Mol Endocrinol 2006;20: 931-43.
44. Hattersley G, Dean T, Gardella TJ. Differential binding selectivity of abaloparatide (BA058) compared to PTH and PTHrP for PTH Type 1 receptor conformations. The Endocrine Society's 96th Annual Meeting 2014.

## Claims

1. A polypeptide of SEQ ID NO:1 and alendronate for use in preventing or reducing non-vertebral bone fractures in a subject in need thereof,
wherein said use comprises administering to the subject the polypeptide once daily by subcutaneous injection for a first period of time of 18 months, followed by an administration of alendronate for six months.

2. The polypeptide of SEQ ID NO:1 for use according to claim 1, wherein the subject has osteoporosis.

3. The polypeptide of SEQ ID NO:1 for use according to claim 1, wherein the alendronate is administered orally.

4. The polypeptide for use according to claim 3 wherein the alendronate is administered at a dose of 5 or 10 mg/day or at a dose of 70 mg/week.

5. The polypeptide for use according to claim 1 wherein the subject has postmenopausal osteoporosis.

6. The polypeptide for use according to claim 1 wherein the subject experiences a risk reduction of fracture for wrist fractures.

7. The polypeptide for use according to claim 1 wherein the subject experiences a risk reduction for hip fractures.

8. The polypeptide for use according to claim 1 wherein the subject experiences an increase in total hip bone mineral density of 2% to 6.5%.

9. The polypeptide for use according to claim 1 wherein the subject experiences an increase in total hip BMD of at least 5.5%.

10. The polypeptide for use according to claim 1 wherein the subject experiences an increase in femoral neck BMD of at least 4.5%.

11. The polypeptide for use according to claim 1, wherein the amount of polypeptide of SEQ ID NO:1 administered is 80 µg.

## Patentansprüche

1. Polypeptid der SEQ ID NR: 1 und Alendronat zur Verwendung beim Verhindern oder Verringern von Frakturen von nicht-vertebralen Knochen bei einem Individuum, das dies benötigt, wobei die Verwendung die Verabreichung des Polypeptids einmal täglich an das Individuum durch subkutane Injektion über einen ersten Zeitraum von 18 Monaten, gefolgt von einer Verabreichung von Alendronat über sechs Monate umfasst.

2. Polypeptid der SEQ ID NO: 1 zur Verwendung nach Anspruch 1, wobei das Individuum Osteoporose aufweist.

3. Polypeptid der SEQ ID NO: 1 zur Verwendung nach Anspruch 1, wobei das Alendronat oral verabreicht wird.

4. Polypeptid zur Verwendung nach Anspruch 3, wobei das Alendronat in einer Dosis von 5 oder 10 mg/Tag oder in einer Dosis von 70 mg/Woche verabreicht wird.

5. Polypeptid zur Verwendung nach Anspruch 1, wobei das Individuum postmenopausale Osteoporose aufweist.

6. Polypeptid zur Verwendung nach Anspruch 1, wobei das Individuum eine Verringerung des Frakturrisikos bei Handgelenksfrakturen erfährt.

7. Polypeptid zur Verwendung nach Anspruch 1, wobei das Individuum eine Verringerung des Risikos bei Hüftfrakturen erfährt.

8. Polypeptid zur Verwendung nach Anspruch 1, wobei das Individuum eine Erhöhung der Gesamt-Knochenmineraldichte der Hüfte von 2 % auf 6,5 % erfährt.

9. Polypeptid zur Verwendung nach Anspruch 1, wobei das Individuum eine Erhöhung der Gesamt-BMD der Hüfte von zumindest 5,5 % erfährt.

10. Polypeptid zur Verwendung nach Anspruch 1, wobei das Individuum eine Erhöhung der Oberschenkel-BMD von zumindest 4,5 % erfährt.

11. Polypeptid zur Verwendung nach Anspruch 1, wobei die Menge des verabreichten Polypeptids der SEQ ID NR:1 80 µg beträgt.

## Revendications

1. Polypeptide de la SEQ ID NO: 1 et alendronate pour une utilisation dans la prévention ou la réduction de fractures osseuses non vertébrales chez un sujet qui en a besoin,
ladite utilisation comprenant l'administration au sujet du polypeptide une fois par jour par injection sous-cutanée pendant une période de temps de 18 mois, suivie par une administration d'alendronate pendant six mois.

2. Polypeptide de la SEQ ID NO: 1 pour une utilisation selon la revendication 1, le sujet étant atteint d'ostéoporose.

3. Polypeptide de la SEQ ID NO: 1 pour une utilisation selon la revendication 1, l'alendronate étant administré oralement.

4. Polypeptide pour une utilisation selon la revendication 3, l'alendronate étant administré à raison d'une dose de 5 ou 10 mg/jour ou à raison d'une dose de 70 mg/semaine.

5. Polypeptide pour une utilisation selon la revendication 1, le sujet étant atteint d'ostéoporose postménopause.

6. Polypeptide pour une utilisation selon la revendication 1, le sujet éprouvant une réduction du risque de fracture pour des fractures du poignet.

7. Polypeptide pour une utilisation selon la revendication 1, le sujet éprouvant une réduction du risque de fractures de la hanche.

8. Polypeptide pour une utilisation selon la revendication 1, le sujet éprouvant une augmentation de la densité minérale osseuse totale de la hanche de 2 % à 6,5 %.

9. Polypeptide pour une utilisation selon la revendication 1, le sujet éprouvant une augmentation de DMO totale de la hanche d'au moins 5,5 %.

10. Polypeptide pour une utilisation selon la revendication 1, le sujet éprouvant une augmentation de DMO du col du fémur d'au moins 4,5 %.

11. Polypeptide pour une utilisation selon la revendication 1, la quantité de polypeptide de la SEQ ID NO: 1 administrée étant de 80 µg.
